# FASCICULE DE BREVET EUROPEEN

(11) **EP 2 430 012 B1**
(45) Date de publication et mention de la délivrance du brevet: **05.11.2014**
(21) Numéro de dépôt: 10728742.7
(22) Date de dépôt: 11.05.2010
(51) Int. Cl.: C07D 401/12, C07D 413/12, C07D 417/12, A61K 31/4523, A61P 29/00, A61P 25/00

(54) **DÉRIVÉS DE 7-AZA-SPIRO[3.5]NONANE-7-CARBOXYLATES, LEUR PRÉPRARATION ET LEUR APPLICATION EN THÉRAPEUTIQUE**
7-AZA-SPIRO[3,5]NONAN-7-CARBOXYLATDERIVATE, DEREN HERSTELLUNG UND DEREN THERAPEUTISCHE VERWENDUNG
7-AZA-SPIRO[3.5]NONANE-7-CARBOXYLATE DERIVATIVES, PREPARATION THEREOF, AND THERAPEUTIC USE THEREOF

(30) Priorité: 12.05.2009 FR 0902269
(43) Date de publication de la demande: 21.03.2012
(73) Titulaire: SANOFI, 75008 Paris (FR)
(72) Inventeur: ABOUABDELLAH, Ahmed, F-75013 Paris (FR); CHEREZE, Nathalie, F-75013 Paris (FR); FAYOL, Aude, F-75013 Paris (FR); LOCHEAD, Alistair, F-75013 Paris (FR); SAADY, Mourad, F-75013 Paris (FR); VACHE, Julien, F-75013 Paris (FR); YAICHE, Philippe, F-75013 Paris (FR)
(74) Mandataire: Le Coupanec, Pascale A.M.P.
(86) Numéro de dépôt international: PCT/FR2010/050914
(87) Numéro de publication internationale: WO 2010/130945

(56) Documents cités:
- WO-A-2005/090322
- WO-A-2005/090347
- WO-A-2006/006490
- US-A1- 2004 167 224

## Description

L'invention a pour objet des dérivés de 7-azaspiro[3.5]nonane-7-carboxylates, leur préparation et leur application en thérapeutique.
Il existe toujours une nécessité de trouver et de développer des produits inhibiteurs de l'enzyme FAAH (Fatty Acide Amide Hydrolase). Les composés de l'invention répondent à ce but.

Les composés de l'invention répondent à la formule générale (I) : 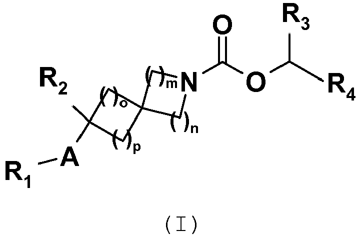 dans laquelle
R₂ représente un atome d'hydrogène, de fluor ou un groupe hydroxyle, cyano, trifluorométhyle, C₁₋₆-alkyle, C₁₋₆-alcoxy, NR₈R₉ ;
m, n, o et p ont pour valeur 1, ou bien, p et o ont pour valeur 1, n et m ont pour valeur 2, ou bien n, o et p ont pour valeur 1 et m a pour valeur 2 ;
A représente un atome d'oxygène;
R₁ représente un groupe R₅ éventuellement substitué par un ou plusieurs groupes R₆ et/ou R₇ ;
R₅ représente un groupe choisi parmi un phényle, pyridinyle, pyridazinyle, pyrimidinyle, pyrazinyle, triazinyle, naphtalènyle, quinolinyle, isoquinolinyle, phtalazinyle, quinazolinyle, quinoxalinyle, cinnolinyle, naphthyridinyle ; R₆ représente un atome d'halogène, un groupe cyano, -CH₂CN, nitro, hydroxyle, C₁₋₆-alkyle, C₁₋₆-alcoxy, C₁₋₆-thioalkyle, C₁₋₆-haloalkyle, C₁₋₆-haloalcoxy, C₁₋₆-halothioalkyle, C₃₋₇-cycloalkyle, C₃₋₇-cycloalkyle- C₁₋₃-alkylène, C₃₋₇-cycloalkyle-C₁₋₃-alkylène-O-, NR₈R₉, NR₈COR₉, NR₈CO₂R₉, NR₈SO₂R₉, NR₈SO₂NR₈R₉, COR₈, CO₂R₈, CONR₈R₉, SO₂R₈, SO₂NR₈R₉ ou - O-(C₁₋3-alkylène)-O- ;
R₇ représente un groupe choisi parmi un phényle, pyridinyle, pyridazinyle, pyrimidinyle, pyrazinyle, triazinyle; le ou les groupes R₇ pouvant être substitués par un ou plusieurs groupes R₆ identiques ou différents l'un de l'autre ;
R₃ représente un atome d'hydrogène, de fluor, un groupe C₁₋₆-alkyle ou un groupe trifluorométhyle ;
R₄ représente un groupe choisi parmi un thiazolyle, un oxazolyle, un isoxazolyle, et un triazolyle;
ce groupe étant éventuellement substitué par un ou plusieurs groupes C₁₋₆-alkyle ou CONR₈R₉ ;
R₈ et R₉ représentent indépendamment l'un de l'autre un atome d'hydrogène ou un groupe C₁₋₆-alkyle,
a l'état de base ou de sel d'addition à un acide.

Parmi les composés de formule générale (I), un premier sous-groupe de composés est constitué des composés pour lesquels R₂ représente un atome d'hydrogène.

Parmi les composés de formule générale (I), un deuxième sous-groupe de composés est constitué des composés pour lesquels m, n, o et p ont pour valeur 1, ou bien, p et o ont pour valeur 1, n et m ont pour valeur 2, ou bien n, o et p ont pour valeur 1 et m a pour valeur 2.

Parmi les composés de formule générale (I), un troisième sous-groupe de composés est constitué des composés pour lesquels R₁ représente un groupe R₅ non substitué ou substitué par un ou plusieurs groupes R₆ et/ou R₇ ;
R₅ représente un groupe phényle, naphtalènyle ou isoquinolinyle ;
R₆ représente un atome d'halogène, plus particulièrement un atome de fluor ou de chlore, ou un groupe C₁₋₆-haloalkyle, plus particulièrement trifluorométhyle, ou un groupe C₁₋₆-alcoxy, plus particulièrement un groupe éthoxy;
R₇ représente un phényle pouvant être substitué par un ou plusieurs groupes R₆ identiques ou différents l'un de l'autre.
Parmi les composés de formule générale (I), un quatrième sous-groupe de composés est constitué des composés pour lesquels R₁ représente un groupe R₅ substitué par un ou plusieurs groupes R₆ et/ou R₇ ;
R₅ représente un groupe phényle;
R₆ représente un atome d'halogène, plus particulièrement un atome de fluor ou de chlore, ou un groupe C₁₋₆-haloalkyle, plus particulièrement trifluorométhyle;
R₇ représente un phényle pouvant être substitué par un ou plusieurs groupes R₆ identiques ou différents l'un de l'autre choisi parmi un atome d'halogène, plus particulièrement un atome de fluor.
Parmi les composés de formule générale (I), un cinquième sous-groupe de composés est constitué des composés pour lesquels R₁ représente un groupe R₅ substitué par un ou plusieurs groupes R₆;
R₅ représente un groupe naphtalèn-2-yle;
R₆ représente un groupe C₁₋₆-alcoxy, plus particulièrement un groupe éthoxy.
Parmi les composés de formule générale (I), un sixième sous-groupe de composés est constitué des composés pour lesquels R₁ représente un groupe R₅ substitué par un ou plusieurs groupes R₆;
R₅ représente un groupe naphtalèn-1-yle;
R₆ représente un atome d'halogène, plus particulièrement un atome de chlore.
Parmi les composés de formule générale (I), un septième sous-groupe de composés est constitué des composés pour lesquels R₁ représente un groupe R₅ non substitué et R₅ représente un groupe isoquinolin-7-yle.

Parmi les composés de formule générale (I), un huitième sous-groupe de composés est constitué des composés pour lesquels R₃ représente un atome d'hydrogène.

Parmi les composés de formule générale (I), un neuvième sous-groupe de composés est constitué des composés pour lesquels R₄ représente un groupe thiazol-4-yle; ce groupe étant non substitué.
Parmi les composés de formule générale (I), un dixième sous-groupe de composés est constitué des composés pour lesquels R₄ représente un groupe thiazol-2-yle;
ce groupe étant substitué par un ou plusieurs groupes CONR₈R₉;
R₈ et R₉ représentent indépendamment l'un de l'autre un atome d'hydrogène ou un groupe C₁₋₆-alkyle. Plus particulièrement, le groupe C₁₋₆-alkyle est un méthyle.
Parmi les composés de formule générale (I), un onzième sous-groupe de composés est constitué des composés pour lesquels R₄ représente un groupe isoxazol-5-yle;
ce groupe étant substitué par un ou plusieurs groupes CONR₈R₉;
R₈ et R₉ représentent indépendamment l'un de l'autre un atome d'hydrogène ou un groupe C₁₋₆-alkyle. Plus particulièrement, le groupe C₁₋₆-alkyle est un méthyle.
Parmi les composés de formule générale (I), un douzième sous-groupe de composés est constitué des composés pour lesquels R₄ représente un groupe choisi parmi un 1H-1,2,4-triazol-5-yle; ce groupe étant substitué par un ou plusieurs groupes C₁₋₆-alkyle.
Parmi les composés de formule générale (I), un treizième sous-groupe de composés est constitué des composés pour lesquels R₄ représente un groupe choisi parmi un oxazol-2-yle;
ce groupe étant substitué par un ou plusieurs groupes CONR₈R₉;
R₈ et R₉ représentent indépendamment l'un de l'autre un atome d'hydrogène ou un groupe C₁₋₆-alkyle. Plus particulièrement, le groupe C₁₋₆-alkyle est un méthyle.
Parmi les composés de formule générale (I), un quatorzième sous-groupe de composés est constitué par les composés de formule générale (I) dans laquelle à la fois R₁ et/ou R₂ et/ou R₃ et/ou R₄ et/ou n et/ou m et/ou o et/ou p et/ou A sont tels que définis dans les groupes ci-dessus.

Parmi les composés de formule générale (I), les composés suivants peuvent être cités (nomenclature IUPAC générée par le logiciel AutoNom):
1. 2-(4-Chloro-phénoxy)-7-aza-spiro[3.5]nonane-7-carboxylate de thiazol-4-ylméthyle
2. 2-(4-chloro-phénoxy)-7-aza-spiro[3.5]nonane-7-carboxylate de 2-méthyl-2H-[1,2,4]triazol-3-ylméthyle et son chlorhydrate
3. 2-(4-Chloro-phénoxy)-7-aza-spiro[3.5]nonane-7-carboxylate de 3-méthylcarbamoyl-isoxazol-5-ylméthyle
4. 2-(4-Chloro-phénoxy)-7-aza-spiro[3.5]nonane-7-carboxylate de 3-carbamoyl-isoxazol-5-ylméthyle
5. 2-(3-Trifluorométhyl-phénoxy)-7-aza-spiro[3.5]nonane-7-carboxylate de 3-méthylcarbamoyl-isoxazol-5-ylméthyle
6. 2-(3-Trifluorométhyl-phénoxy)-7-aza-spiro[3.5]nonane-7-carboxylate de 3-carbamoyl-isoxazol-5-ylméthyle
7. 2-(4'-Fluoro-biphényl-3- yloxy)-7-azaspiro[3.5]nonane-7-carboxylate de 3-méthylcarbamoyl-isoxazol-5-ylméthyle
8. 2-(7-Ethoxy-naphthalèn-2-yloxy)-7-aza-spiro[3.5]nonane-7-carboxylate de 3-méthylcarbamoyl-isoxazol-5-ylméthyle
9. 2-(3-trifluorométhyl-phénoxy)-6-aza-spiro[3.4]octane-6-carboxylate de 3-carbamoyl-isoxazol -5-ylméthyle et son chlorhydrate (isomères I + II)
10. 2-(3-Trifluorométhyl-phénoxy)-6-aza-spiro[3.4]octane-6-carboxylate de 3-carbamoyl-isoxazol-5-ylméthyle (isomère I du composé n°9)
11. 2-(3-Trifluorométhyl-phénoxy)-6-aza-spiro[3.4]octane-6-carboxylate de 3-carbamoyl-isoxazol-5-ylméthyle (isomère II du composé n°9)
12. 2-(4'-Fluoro-biphényl-3-yloxy)-7-aza-spiro[3.5]nonane-7-carboxylate de 3-carbamoyl-isoxazol-5-ylméthyle
13. 2-(7-Ethoxy-naphthalèn-2-yloxy)-7-aza-spiro[3.5]nonane-7-carboxylate de 3-carbamoyl-isoxazol-5-ylméthyle
14. 2-(3-Trifluorométhyl-phénoxy)-6-aza-spiro[3.4]octane-6-carboxylate de 4-carbamoyl-oxazol-2-ylméthyle (isomères I + II)
15. 2-(3-Trifluorométhyl-phénoxy)-6-aza-spiro[3.4]octane-6-carboxylate de 4-méthylcarbamoyl-oxazol-2-ylméthyle (isomères I + II)
16. 2-(4-Chloro-naphthalèn-1-yloxy)-7-aza-spiro[3.5]nonane-7-carboxylate de 3-carbamoyl-isoxazol-5-ylméthyle
17. 2-(4-Chloro-naphthalèn-1-yloxy)-7-azaspiro[3.5]nonane-7-carboxylate de 3-méthylcarbamoyl-isoxazol-5-ylméthyle
18. 2-(4'-Fluoro-biphényl-4-yloxy)-7-azaspiro[3.5]nonane-7-carboxylate de 3-carbamoyl-isoxazol-5-ylméthyle
19. 2-(4'-Fluoro-biphényl-4-yloxy)-7-azaspiro[3.5]nonane-7-carboxylate de 3-méthylcarbamoylisoxazol-5-ylméthyle
20. 2-(4'-Fluoro- biphényl-3-yloxy)-7-azaspiro[3.5]nonane-7-carboxylate de 4-méthylcarbamoylthiazol-2-ylméthyle
21. 2-(Isoquinolin-7-yloxy)-7-aza-spiro[3.5]nonane-7-carboxylate de 3-méthylcarbamoyl-isoxazol-5-ylméthyle
22. 2-(4-Chloro-3-fluoro-phénoxy)-7-azaspiro[3.5]nonane-7-carboxylate de 3-méthylcarbamoyl-isoxazol-5-ylméthyle
23. 6-(4-Chloro-3-fluoro-phénoxy)-2-azaspiro[3.3]heptane-2-carboxylate de 3-méthylcarbamoyl-isoxazol-5-ylméthyle
24. 6-(4-Chloro-3-fluoro-phénoxy)-2-azaspiro[3.3]heptane-2-carboxylate de 3-carbamoyl-isoxazol-5-ylméthyle
25. 6-(4'-Fluoro-biphényl-4-yloxy)-2-azaspiro[3.3]heptane-2-carboxylate de 3-méthylcarbamoyl-isoxazol-5-ylméthyle
26. 2-(4-Chloro-3-fluoro-phénoxy)-6-azaspiro[3.4]octane-6-carboxylate de 3-carbamoyl-isoxazol-5-ylméthyle (isomères I + II)
27. 2-(4-Chloro-phénoxy)-6-aza-spiro[3.4]octane-6-carboxylate de 3-méthylcarbamoyl-isoxazol-5-ylméthyle (isomères I + II)
28. 2-(4'-Fluoro-biphényl-4-yloxy)-6-azaspiro[3.4]octane-6-carboxylate de 3-carbamoyl-isoxazol-5-ylméthyle (isomères I + II)
29. 2-(4'-Fluoro-biphényl-4-yloxy)-6-azaspiro[3.4]octane-6-carboxylate de 3-méthylcarbamoyl-isoxazol-5-ylméthyle (isomères I + II)
30. 2-(4-Chloro-naphthalèn-1-yloxy)-6-azaspiro[3.4]octane-6-carboxylate de 3-carbamoyl-isoxazol-5-ylméthyle (isomères I + II)
31. 2-(4-Chloro-naphthalèn-1-yloxy)-6-azaspiro[3.4]octane-6-carboxylate de 3-méthylcarbamoylisoxazol-5-ylméthyle (isomères I + II)
32. 2-(4'-Fluoro-biphényl-4-yloxy)-6-azaspiro[3.4]octane-6-carboxylate de 3-méthylcarbamoyl-isoxazol-5-ylméthyle(isomère I du compose 29)
33. 2-(4'-Fluoro- biphényl-4-yloxy)-6-azaspiro[3.4]octane-6-carboxylate de 3-méthylcarbamoyl-isoxazol-5-ylméthyle(isomère II du composé 29).

Les composés de formule générale (I) peuvent comporter un ou plusieurs carbones asymétriques. Ils peuvent exister sous forme d'énantiomères ou de diastéréoisomères. Les composés de formule générale (I) peuvent également exister sous forme de stéréoisomères *cis* ou *trans.* Ces stéréoisomères, énantiomères et diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Dans le cadre de l'invention, on entend par :
- C_{t-z} où t et z peuvent prendre les valeurs de 1 à 8, une chaîne carbonée pouvant avoir de t à z atomes de carbone, par exemple C₁₋₃ une chaîne carbonée qui peut avoir de 1 à 3 atomes de carbone ;
- alkyle, un groupe aliphatique saturé, linéaire ou ramifié; par exemple un groupe C₁₋₆-alkyle représente une chaîne carbonée de 1 à 6 atomes de carbone, linéaire ou ramifiée, plus particulièrement un méthyle, éthyle, propyle, isopropyle, butyle, isobutyle, tert-butyle, pentyle, hexyle ;
- alkylène, un groupe alkyle divalent saturé, linéaire ou ramifié, par exemple un groupe C₁₋₃-alkylène représente une chaîne carbonée divalente de 1 à 3 atomes de carbone, linéaire ou ramifiée, plus particulièrement un méthylène, éthylène, 1-méthyléthylène, propylène ;
- cycloalkyle, un groupe alkyle cyclique, par exemple un groupe C₃₋₇-cycloalkyle représente un groupe carboné cyclique de 3 à 7 atomes de carbone, plus particulièrement un cyclopropyle, cyclobutyle, cyclopentyle, cyclohexyle, cycloheptyle ;
- alcoxy, un groupe -O-alkyle à chaîne aliphatique saturée, linéaire ou ramifiée ;
- thioalkyle, un groupe -S-alkyle à chaîne aliphatique saturée, linéaire ou ramifiée ;
- haloalkyle, un groupe alkyle dont un ou plusieurs atomes d'hydrogène ont été substitués par un atome d'halogène ;
- haloalcoxy, un groupe alcoxy dont un ou plusieurs atomes d'hydrogène ont été substitués par un atome d'halogène ;
- halothioalkyle, un groupe thioalkyle dont un ou plusieurs atomes d'hydrogène ont été substitués par un atome d'halogène ;
- atome d'halogène, un fluor, un chlore, un brome ou un iode
- TFA : acide trifluoroacétique ;
- ACN : acétonitrile.

Les composés de l'invention peuvent être préparés selon différentes méthodes, illustrées par les schémas qui suivent. Ces méthodes, ainsi que les composés intermédiaires utilisés, sont un objet de la présente invention.

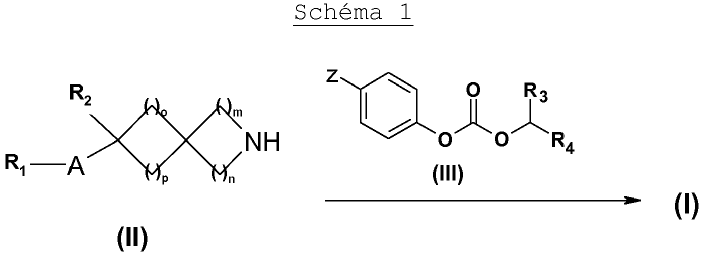

Ainsi une première méthode de préparation (schéma 1) consiste à faire réagir une amine de formule générale (II), dans laquelle A, R₁, R₂, m, n, o et p sont tels que définis dans la formule générale (I) définie ci-dessus, avec un carbonate de formule générale (III) dans laquelle Z représente un atome d'hydrogène ou un groupe nitro, R₃ et R₄ sont tels que définis dans la formule générale (I) définie ci-dessus, en présence d'une base telle que la triéthylamine, la pyridine, la *N,N*-diméthylaminopyridine ou la *N,N*-diisopropyléthylamine, dans un solvant tel que le toluène, l'acétonitrile ou le dichloroéthane, à une température comprise entre la température ambiante et la température de reflux du solvant.

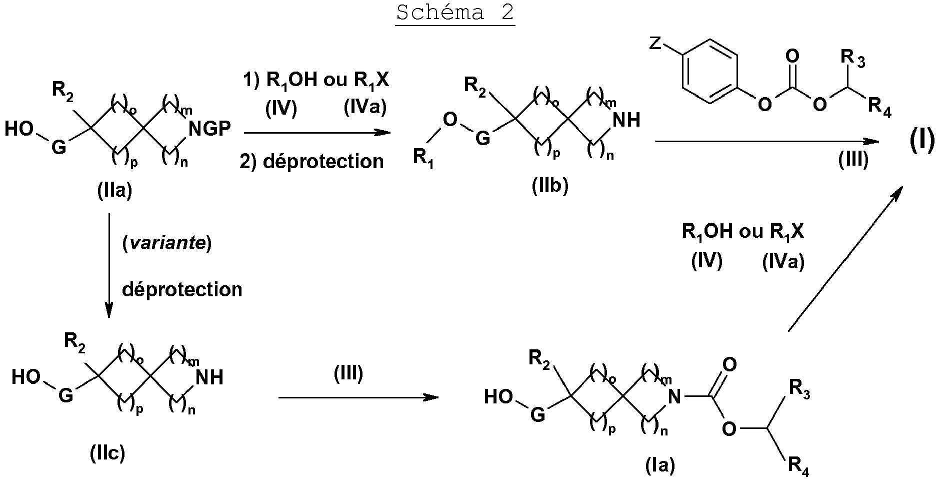

Une seconde méthode de préparation (schéma 2) pour obtenir les composés de formule générale (I) dans laquelle A représente plus particulièrement un atome d'oxygène, consiste à faire réagir, dans un premier temps, un alcool de formule générale (IIa), dans laquelle R₂, m, n, o et p sont tels que définis dans la formule générale (I) définie ci-dessus, G représente une partie du groupe A tel que défini dans la formule générale (I) à savoir une liaison covalente, et GP représente un groupe protecteur tel qu'un Boc (*tert-*butyloxycarbonyl), un Cbz (benzyloxycarbonyl), un benzyle ou un benzhydrile ;
- soit avec un dérivé alcool de formule générale (IV), dans laquelle R₁ est tel que défini ci-dessus en utilisant les conditions de réaction de Mitsunobu *(*Synthesis, 1981, 1-28),
- soit avec un dérivé halogéné de formule générale (IVa), dans laquelle R₁ est tel que défini ci-dessus et X représente un atome de fluor, de chlore, de brome ou d'iode en utilisant les réactions de substitution nucléophiles aromatiques, hétéroaromatiques ou de *O-*arylation, O-hétéroarylation de Buchwald, par exemple
   au moyen d'un catalyseur au Palladium ou au Cuivre ; suivie d'une réaction de déprotection par exemple en présence d'acide trifluoroacétique ou d'une solution d'acide chlorhydrique dans l'isopropanol ou le dioxane, pour conduire à l'amine de formule générale (IIb) dans laquelle G, R₂, m, n, o et p sont tels que définis dans l'amine de formule (IIa) ci-dessus et R₁ est tel que défini dans la formule générale (I) définie ci-dessus. Une alternative à la réaction de Mitsunobu consiste à faire réagir un dérivé alcool de formule générale (IV) avec les composés de formule générale (IIe) obtenus par activation de la fonction alcool des composés de formule générale (IIa) par un groupe tosylate. Le dérivé de formule générale (IIb) ainsi obtenu est ensuite transformé en composé de formule générale (I) selon une réaction de condensation avec un carbonate de formule générale (III) telle que définie ci-dessus, dans les conditions décrites ci-dessus (schéma 1).

Une variante d'obtention des composés de formule générale (I) (schéma 2) dans laquelle A représente plus particulièrement un atome d'oxygène, consiste à déprotéger un alcool de formule générale (IIa) tel que défini ci-dessus, selon une réaction de déprotection telle que définie ci-dessus afin d'obtenir un aminoalcool de formule générale (IIc) puis à faire réagir cet aminoalcool de formule générale (IIc) dans laquelle R₂, m, n, o et p sont tels que définis dans la formule générale (I) définie ci-dessus, et G représente une partie du groupe A tel que défini dans la formule générale (I), à savoir une liaison covalente, avec un carbonate de formule générale (III) telle que définie ci-dessus, dans les conditions décrites ci-dessus (schéma 1), pour conduire au dérivé carbamate de formule générale (Ia), dans laquelle R₂, R₃, R₄, m, n, o et p sont tels que définis dans la formule générale (I) définie ci-dessus et G représente une partie du groupe A tel que défini dans la formule générale (I), à savoir une liaison covalente. Le dérivé carbamate (Ia) ainsi obtenu est ensuite transformé en composé de formule générale (I), par action d'un alcool de formule générale R₁OH (IV) telle que définie ci-dessus, en utilisant les conditions de réaction de Mitsunobu ou par action d'un dérivé halogéné de formule générale R₁X (IVa) tel que défini ci-dessus en utilisant les réactions de substitution nucléophiles aromatiques, hétéroaromatiques ou de O-arylation, O-hétéroarylation de Buchwald, par exemple au moyen d'un catalyseur au Palladium ou au Cuivre.

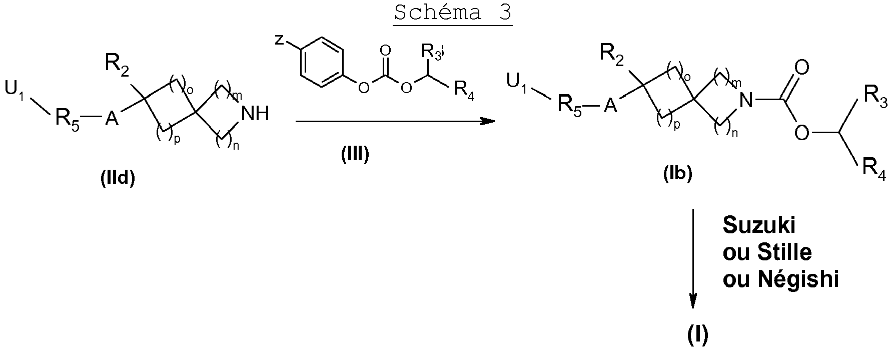

Une troisième méthode (schéma 3) a été développée pour ce qui concerne la synthèse des composés de formule générale (I), dans laquelle R₁ représente un groupe R₅ substitué notamment par un groupe R₆ de type C₁₋₆-alkyle, C₃₋₇-cycloalkyle ou C₃₋₇-cycloalkyle-C₁₋₃-alkylène, ou par un groupe R₇ tel que défini dans la formule générale (I) définie ci-dessus. Ainsi, la première étape consiste à faire réagir une amine de formule générale (IId), dans laquelle A, R₂, R₅, m, n, o et p sont tels que définis dans la formule générale (I) définie ci-dessus et U₁ représente un atome de chlore, de brome, d'iode ou un groupement triflate, avec un carbonate de formule générale (III) telle que définie ci-dessus, dans les conditions décrites ci-dessus (schéma 1), pour conduire au dérivé carbamate de formule générale (Ib), dans laquelle A, R₂, R₃, R₄, R₅, m, n, o et p sont tels que définis dans la formule générale (I) définie ci-dessus et U₁ est tel que défini ci-dessus. La réaction de couplage catalysée au moyen d'un métal de transition comme le Palladium (0) est ensuite réalisée sur l'intermédiaire clé de formule générale (Ib) telle que définie ci-dessus, U₁ étant dans la position où l'on souhaite introduire le groupe R₆ ou R₇ (schéma 3):
- soit par une réaction de type Suzuki, par exemple au moyen d'un acide boronique d'alkyle, de cycloalkyle, d'aryle ou d'hétéroaryle,
- soit selon une réaction de type Stille, par exemple en utilisant un dérivé tri-alkylstanneux d'aryle ou d'hétéroaryle
- soit par une réaction de type Negishi, par exemple en utilisant un dérivé zincate d'halogénure d'alkyle, de cycloalkyle, d'aryle ou d'hétéroaryle.

Un autre objet de la présente invention se rapporte aux composés de formule (III) 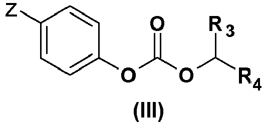 dans laquelle Z représente un atome d'hydrogène ou un groupe nitro, R₃ est tel que défini dans la formule (I) et R₄ représente un groupe 4-(méthylcarbamoyl)-oxazol-2-yle.

Un autre objet de la présente invention se rapporte aux composés de formule générale (Ia) : 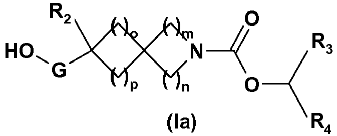 dans laquelle R₂, R₃, R₄, m, n, o et p sont tels que définis dans la formule générale (I), et G représente une partie du groupe A tel que défini dans la formule générale (I), à savoir une liaison covalente.
Parmi ces composés, on peut citer :
2-Hydroxy-7-aza-spiro[3.5]nonane-7-carboxylate de thiazol-4-ylméthyle.

Un autre objet de la présente invention se rapporte aux composés de formule générale (Ib) 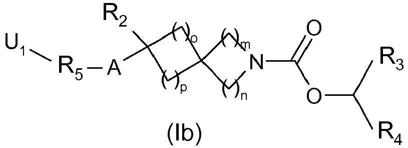 dans laquelle A, R₂, R₃, R₄, R₅, m, n, o et p sont tels que définis dans la formule générale (I) selon la revendication 1 et U₁ représente un atome de chlore, de brome, d'iode ou un groupement triflate.

Un autre objet de la présente invention se rapporte aux composés de formule générale (II), 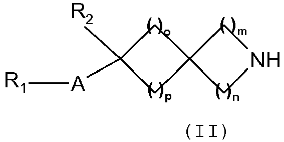 dans laquelle R₁, R₂, m, n, o et p sont tels que définis dans la formule générale (I), A représente un atome d'oxygène, étant donné que R₁ n'est pas un groupe fluorophényl.
Parmi ces composés, on peut citer :
2-((4-Chloro-phénoxy)-7-aza-spiro[3.5]nonane ;
2-((4'-Fluoro-biphényl-3-yloxy)-7-aza-spiro[3.5]nonane;
2-((4'-Fluoro-biphényl-4-yloxy)-7-aza-spiro[3.5]nonane (RMN-1H (DMSO) δ (ppm) : 8,80 (s large, 2H); 7,65 (t, 2H); 7,60 (d, 2H), 7,30 (t, 2H); 6,95 (d, 2H); 4,80 (qt, 1H); 3,00 (d large, 4H); 2,55 (m, 2H); 1,90 (m, 2H); 1,80 (dt, 4H));
2-((4-chloro-naphthalèn-1-yloxy)-7-aza-spiro[3.5]nonane;
2-((4-chloro-naphthalèn-1-yloxy)-6-aza-spiro[3.4]octane;
2-((3-Trifluorométhyl-phénoxy)-6-aza-spiro[3.4]octane;
2-((4'-fluoro-biphényl-4-yloxy)-6-aza-spiro[3.4]octane;
6-((4-chloro-3-fluoro-phénoxy)-2-aza-spiro[3.3]heptane;
6-((4'-fluoro-biphényl-4-yloxy)-2-aza-spiro[3.3]heptane;
2-((7-éthoxy-naphthalèn-2-yloxy)-7-aza-spiro[3.5]nonane (RMN-1H (DMSO) δ (ppm) : 8,90 (s large, 2H); 7,75 (d, 2H); 7,20 (s, 1H); 7,10 (s, 1H); 7,00 (d, 1H); 6,95 (d, 1H); 4,85 (qt, 1H); 4,15 (qd, 2H); 3,00 (d large, 4H); 2,55 (m, 2H); 1,95 (m, 2H); 1,80 (dt, 4H); 1,40 (t, 3H));
6-((7-Aza-spiro[3.5]non-2-yloxy)-isoquinoline (RMN-1H (DMSO) δ (ppm) : 9,20 (s, 1H); 8,35 (d, 1H); 7,90 (d, 1H) ; 7, 65 (d, 1H), 7,40 (t, 2H) ; 4,90 (qt, 1H) ; 2,65 (d large, 4H); 2,50 (m, 2H); 1,85 (m, 2H); 1,55 (dt, 4H));
2-((4-chloro-phénoxy)-6-aza-spiro[3.4]octane (RMN-1H (DMSO) δ (ppm): 8,80 (s large, 2H); 7,35 (d, 1H) ; 6,90 (t, 2H) ; 4,75 (qt, 1H); 3,20 (m, 4H) ; 2,60 (m, 2H); 2,15 (m, 2H); 2,00 (m, 2H)).

Un autre objet de la présente invention se rapporte aux composés de formule générale (IIa), 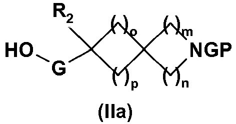 dans laquelle R₂ est tel que défini dans la formule générale (I), o et p représentent 1, m et n représentent 1 ou 2, mais m et n ne réprésentent pas ensemble la valeur 2, G représente une partie du groupe A tel que défini dans la formule générale (I), à savoir une liaison covalente, et GP représente un groupe protecteur tel qu'un Boc (tert-butyoxyocarbonyl), un Cbz (benzyloxycarbonyl, un benzyle ou un benzhydryle.
Parmi ces composés, on peut citer :
2-Hydroxy-6-aza-spiro[3.4]octane-6-carboxylate de tert-butyle
   (RMN 1H (DMSO) δ (ppm) : 4,7 (t, 1H) ; 4,1 (m, 1H) ; 3,2 (m, 4H), ; 2,2 (m, 2H) ; 1, 8 (m, 4H) ; 1,4 (s, 9H)) ; 6-Hydroxy-2-aza-spiro[3.3]heptane-2-carboxylate de tert-butyle
   (RMN-1H (DMSO) δ (ppm): 5,00 (d, 1H) ; 3,95 (hex, 1H); 3,75 (d, 4H); 2,40 (m, 2H); 1,95 (m, 2H); 1,40 (s, 9H)).

Un autre objet de la présente invention se rapporte aux composés de formule générale (IIe), 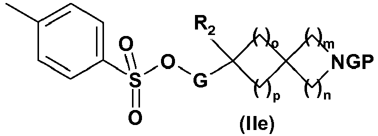 dans laquelle R₂ est tel que défini dans la formule générale (I), o et p représentent 1, m et n représentent 1 ou 2, mais m et n ne réprésentent pas ensemble la valeur 2, G représente une partie du groupe A tel que défini dans la formule générale (I), à savoir une liaison covalente, et GP représente un groupe protecteur tel qu'un Boc (tert-butyoxyocarbonyl), un Cbz (benzyloxycarbonyl, un benzyle ou un benzhydryle.
Parmi ces composés, on peut citer :
2-((Toluène-4-sulfonyloxy)-6-aza-spiro[3.4]octane-6-carboxylate de tert-butyle
   (RMN-1H (DMSO) δ (ppm) : 7,80 (d, 2H); 7,50 (d, 2H); 4,90 (m, 1H); 3,15 (m, 4H); 2,45 (s, 3H); 2,20 (m, 2H); 2,10 (m, 2H); 1,80 (t, 2H); 1,40 (s, 9H)) ;
2-((Toluène-4-sulfonyloxy)-6-aza-spiro[3.4]octane-6-carboxylate de tert-butyle (isomère 2a)
   (RMN-1H (DMSO) δ (ppm) : 7,80 (d, 2H); 7,50 (d, 2H); 4,90 (qt, 1H); 3,15 (m, 4H); 2,45 (s, 3H); 2,25 (t, 2H); 2,05 (t, 2H) ; 1,80 (m, 2H) ; 1,40 (s, 9H)) ;
2-((Toluène-4-sulfonyloxy)-6-aza-spiro[3.4]octane-6-carboxylate de tert-butyle (isomère 2b)
   (RMN 1H (DMSO) δ (ppm) : 7,80 (d, 2H) ; 7,50 (d, 2H) ; 4,90 (qt, 1H); 3,20 (m, 4H); 2,45 (s, 3H); 2,20 (t, 2H); 2,10 (t, 2H); 1,80 (m, 2H); 1,40 (s, 9H)).

Les autres composés de formules générales (II), (IIa), (IIb), (IIc), (IId), (III), (IV) et (IVa) ainsi que les autres réactifs sont disponibles dans le commerce ou décrits dans la littérature, ou bien peuvent être préparés selon des méthodes qui y sont décrites ou qui sont connues de l'homme du métier.

Les exemples qui suivent illustrent la préparation de quelques composés de l'invention. Ces exemples ne sont pas limitatifs et ne font qu'illustrer l'invention. Les microanalyses, les spectres I.R. et R.M.N. et/ou la LC-MS (Liquid Chromatography coupled to Mass Spectroscopy) confirment les structures et les puretés des composés obtenus.
PF(°C) représente le point de fusion en degrés Celsius.
R_{f} indique le temps de rétention obtenu par analyse CCM (Chromatographie sur Couche Mince).
Les numéros indiqués entre parenthèses dans les titres des exemples correspondent à ceux de la 1ère colonne des tableaux ci-après.

La nomenclature UICPA (Union Internationale de Chimie Pure et Appliquée - IUPAC en anglais) a été utilisée pour la dénomination des composés dans les exemples ci-dessous.
Le composé n°9 est un mélange d'isomères. Le composé n°10 est l'isomère I du composé n°9 et le composé n° 11 est l'isomère II du composé n°9.
Le composé n°29 est un mélange d'isomères. Le composé n°32 est l'isomère I du composé n°29 et le composé n° 33 est l'isomère II du composé n°29.
Les composés 14, 15, 26, 27, 28, 30 et 31 sont des mélanges d'isomères.

### Exemple 1 (Composé N°1)

### 2-(4-Chloro-phénoxy)-7-aza-spiro[3.5]nonane-7-carboxylate de thiazol-4-ylméthyle

### 1.1. Acétate de 7-aza-spiro[3.5]non-2-yle, bromhydrate

0,800 g (2,91 mmoles) de 2-hydroxy-7-aza-spiro[3.5]nonane-7-carboxylate de benzyle (WO 9222550) est lentement ajouté sur 5 mL d'une solution d'acide bromhydrique à 5,7 N dans l'acide acétique refroidie à 0°C. Après 1 heure d'agitation à 0°C, 50 mL d'éther diéthylique sont ajoutés et le milieu est agité pendant 1 heure. Le précipité formé est filtré sur fritté et abondamment rincé à l'éther diéthylique. Après séchage sous vide 1 nuit à 80°C, on obtient 0,380 g de produit attendu sous la forme d'un solide blanc.

### 1.2. 2-Acétoxy-7-aza-spiro[3.5]nonane-7-carboxylate de thiazol-4-ylméthyle

0,280 g (1,06 mmoles) d'acétate de 7-aza-spiro[3.5]non-2-yle, bromhydrate, obtenu à l'étape 1.1, est dissous dans 3 ml de méthanol. 0,20 mL (1,17 mmoles) de N,N-diisopropyléthylamine est ajouté à température ambiante. Le milieu est agité 3 minutes puis 0,297 g (1,06 mmoles) de (4-nitro-phényle)- carbonate de thiazole-4-ylméthyle (WO2008013834) en solution dans 3 mL de dichlorométhane est ajouté. Après 14 heures d'agitation à température ambiante, le milieu est dilué avec du dichlorométhane et une solution aqueuse de soude à 1N. Après séparation de la phase aqueuse, la phase organique est lavée deux fois avec une solution aqueuse de soude à 1N puis trois fois avec une solution aqueuse saturée en chlorure d'ammonium, séchée sur sulfate de sodium, filtrée et concentrée à sec. On obtient 0.33 g du produit attendu sous la forme d'une poudre, utilisée telle quelle dans l'étape suivante.
Point de fusion (°C) = 94-96°C
RMN-¹H (CDCl3) δ (ppm) : 8,85 (s, 1H); 7,40 (s, 1H); 5,35 (s, 2H); 5,10 (qt, 1H); 3,50 (m, 4H); 2,40 (m, 2H); 2,10 (s, 3H); 1,90 (m, 2H); 1,65 (m, 4H).

### 1.3. 2-Hydroxy-7-aza-spiro[3.5]nonane-7-carboxylate de thiazol-4-ylméthyle

0,267 g (0,82 mmole) du 2-acétoxy-7-aza-spiro[3.5]nonane-7-carboxylate de thiazol-4-ylméthyle, obtenu à l'étape 1.2., est dissous dans 2 mL de méthanol puis 0,5 mL d'eau et 0,114 g (0,82 mmole) de carbonate de potassium sont ajoutés. Après 1 heure d'agitation à température ambiante, le milieu est concentré sous vide puis repris avec de l'eau. La solution aqueuse est extraite deux fois au dichlorométhane puis les phases organiques sont réunies, séchées sur sulfate de sodium, filtrées et concentrées à sec. On obtient 0,222 g du produit attendu sous la forme d'une huile incolore.
RMN-¹H (CDCl₃) δ (ppm) : 8,75 (s, 1H); 7,30 (s, 1H); 5,20 (s, 2H); 4,25 (qt, 1H); 3,35 (m, 4H); 2,20 (m, 2H); 1,65 (m, 2H); 1,45 (m, 4H).

### 1.4. 2-(4-Chloro-phénoxy)-7-aza-spiro[3.5]nonane-7-carboxylate de thiazol-4-ylméthyle

Sous atmosphère inerte, 0,11 g (0,39 mmole) de 2-hydroxy-7-aza-spiro[3.5]nonane-7-carboxylate de thiazol-4-ylméthyle, obtenu à l'étape 1.3., est mis en solution dans 4 mL de toluène. 0,160 g (0,61 mmole) de triphénylphosphine et 0,060 g (0,47 mmole) de 4-chlorophénol sont ajoutés. Le milieu est refroidi à 0°C et une solution de 0,096 g (0,48 mmole) de diéthylazodicarboxylate dans 1 mL de toluène est additionnée. Le milieu est agité 14 heures à température ambiante puis concentré sous vide. Le résidu obtenu est repris dans l'eau et extrait deux fois avec de l'acétate d'éthyle. Les phases organiques groupées sont séchées sur sulfate de sodium, filtrées et concentrées sous vide. Le résidu obtenu est purifié par chromatographie sur gel de silice en éluant avec un mélange de 50 /50 à 80/20 de cyclohexane et d'acétate d'éthyle. On obtient 0,04 g de produit attendu sous la forme d'un solide.
Point de fusion (°C) : 86-88°C
LC-MS : M+H = 393
RMN-¹H (CDCl3) δ (ppm) : 8,85 (s, 1H); 7,40 (s, 1H); 7,30 (t, 2H); 6,80 (d, 2H); 5,35 (s, 2H); 4,70 (qt, 1H); 3,35 (m, 4H); 2,45 (m, 2H); 2,00 (m, 2H); 1,65 (m, 4H).

### Exemple 2 (Composé N°2)

### Chlorhydrate de 2-(4-chloro-phénoxy)-7-aza-spiro[3.5]nonane-7-carboxylate de 2-méthyl-2H-[1,2,4]triazol-3-ylméthyle

### 2.1. 2-(4-Chloro-phénoxy)-7-aza-spiro[3.5]nonane-7-carboxylate de tert-butyle

Sous atmosphère inerte, 0,60 g (2,49 mmoles) de 2-hydroxy-7-aza-spiro[3.5]nonane-7-carboxylate de tert-butyle (WO 2003084948), est mis en solution dans 25 mL de toluène. 1,024 g (3,90 mmoles) de triphénylphosphine et 0,384 g (2,98 mmoles) de 4-chlorophénol sont ajoutés. Le milieu est refroidi à 0°C puis une solution de 0,528 g (3,03 mmoles) de diéthylazodicarboxylate dans 3 mL de toluène est additionnée. Le milieu est agité 14 heures à température ambiante puis concentré sous vide. Le résidu obtenu est repris avec une solution aqueuse saturée en carbonate de sodium et extrait deux fois au dichlorométhane. Les phases organiques groupées sont lavées une fois avec une solution aqueuse saturée en chlorure de sodium, séchées sur sulfate de sodium, filtrées et concentrées sous vide. On obtient 0,874 g d'un résidu utilisé tel quel dans l'étape suivante.

### 2.2. 2-(4-Chloro-phénoxy)-7-aza-spiro[3.5]nonane

0,874 g (2,49 mmoles) de 2-(4-chloro-phénoxy)-7-azaspiro[3.5]nonane-7-carboxylate de tert-butyle, obtenu à l'étape 2.1., est repris dans 5 mL de dioxane et 9,32 mL (37,29 mmoles) d'une solution 4N d'acide chlorhydrique dans le dioxane sont ajoutés lentement sous agitation. Après 3 heures d'agitation à température ambiante, le milieu est concentré sous vide et le résidu est repris dans une solution aqueuse 1N d'acide chlorhydrique. La phase aqueuse est extraite deux fois à l'acétate d'éthyle puis lentement alcalinisée jusqu'à pH 10 par addition de soude à 35%. La phase aqueuse est extraite trois fois au dichlorométhane. Ces trois extraits organiques sont groupés, lavés une fois avec une solution aqueuse saturée en chlorure de sodium, séchés sur sulfate de sodium, filtrés et concentrés sous vide. On obtient 0,460 g de produit attendu sous la forme d'une huile jaune.
RMN-¹H (DMSO) δ (ppm) : 7,30 (d, 2H) ; 6,85 (d, 2H) ; 4,70 (qt, 1H) ; 2,65 (dt, 4H) ; 2,40 (m, 2H) ; 1,75 (m, 2H) ; 1,50 (dt, 4H).

### 2.3. 2-(4-Chloro-phénoxy)-7-aza-spiro[3.5]nonane-7-carboxylate de 2-méthyl-2H-[1,2,4]triazol-3-ylméthyle

0,100 g (0,40 mmole) de (2-méthyl-2H-[1,2,4]triazol-3-yl)-méthanol et 0,15 mL (0,83 mmole) de N,N-diisopropyléthylamine sont mis en solution dans 4 mL de 1,2-dichloroéthane. Le milieu est refroidi à 0°C puis 0,08 g (0,40 mmole) de chloroformiate de p-nitrophényle en solution dans 2 mL de 1,2-dichloroéthane est additionné. Le mélange est agité à température ambiante pendant 15 minutes puis 0,100 g (0,40 mmole) de 2-(4-chloro-phénoxy)-7-azaspiro[3.5]nonane, obtenu à l'étape 2.2., est ajouté. Le mélange est chauffé à 60°C pendant 15 heures. Après retour à température ambiante, le milieu est dilué avec une solution aqueuse de soude à 1N, et le produit est extrait avec du dichlorométhane. Les phases organiques réunies sont ensuite successivement lavées trois fois par une solution aqueuse de soude à 1M, deux fois par une solution aqueuse saturée de chlorure d'ammonium, une fois par une solution aqueuse saturée de chlorure de sodium, séchées sur sulfate de sodium, filtrées et évaporées à sec. Après purification sur colonne de gel de silice en éluant avec du dichlorométhane puis avec un mélange 99/1/0,1 puis 98/2/0,2 et 97/3/0,3 de dichlorométhane, de méthanol et d'ammoniaque à 30%, on obtient 0,100 g de produit attendu sous la forme d'une huile incolore qui est reprise dans une solution d'acide chlorhydrique à 5-6 N dans le propan-2-ol. Le solide blanc formé est repris par de l'éther diéthylique, filtré, et séché sous vide. On obtient 0,80 g du chlorhydrate du produit attendu sous la forme d'une poudre blanche.
Point de fusion (°C) : 163-165°C
LC-MS : M+H = 391
RMN-¹H (DMSO) δ (ppm) : 7,95 (s, 1H); 7,30 (d, 2H); 6,85 (d, 2H) ; 5,20 (s, 2H) ; 4, 75 (qt, 1H) ; 3,90 (s, 3H) ; 3, 35 (d large, 4H); 2,45 (m, 2H); 1,80 (m, 2H); 1,55 (dt, 4H).

### Exemple 3 (Composé N°3)

### 2-(4-Chloro-phénoxy)-7-aza-spiro[3.5]nonane-7-carboxylate de 3-méthylcarbamoyl-isoxazol-5-ylméthyle

### 3.1 2-(4-Chloro-phénoxy)-7-aza-spiro[3.5]nonane-7-carboxylate de 3-éthoxycarbonyl-isoxazol-5-ylméthyle

0,172 g (1,00 mmole) de 5-hydroxyméthyl-isoxazole-3-carboxylate d'éthyle et 0,33 mL (1,92 mmoles) de N,N-diisopropyléthylamine sont mis en solution dans 9 mL de 1,2-dichloroéthane puis refroidis à 0°C. 0,184 g (0,91 mmole) de chloroformiate de p-nitrophényle en solution dans 2 mL de 1,2-dichloroéthane sont additionnés. Le mélange est agité à température ambiante pendant 20 minutes puis 0,230 g (0,91 mmole) de 2-(4-chloro-phénoxy)-7-aza-spiro[3.5]nonane, obtenu à l'étape 2.2., est ajouté. Le mélange est chauffé à 60°C pendant 15 heures. Après retour à température ambiante, une solution aqueuse de soude à 1N est ajoutée, et le produit est extrait avec du dichlorométhane. Les phases organiques réunies sont ensuite successivement lavées trois fois par une solution aqueuse de soude à 1N, deux fois par une solution aqueuse saturée de chlorure d'ammonium, une fois par une solution aqueuse saturée de chlorure de sodium, séchées sur sulfate de sodium, filtrées et évaporées à sec. On obtient 0,447 g de produit attendu sous la forme d'une huile incolore qui est utilisée telle quelle dans l'étape suivante.

### 3.2. 2-(4-Chloro-phénoxy)-7- aza-spiro[3.5]nonane-7-carboxylate de 3-méthylcarbamoyl-isoxazol-5-ylméthyl

Dans un tube scellé, 0,180 g (0,40 mmole) de 2-(4-chlorophénoxy)-7-aza-spiro[3.5]nonane-7-carboxylate de 3-éthoxycarbonyl-isoxazol-5-ylméthyle, obtenu à l'étape 3.1., est mis en solution dans 5 mL de méthanol. 0,5 mL (4,00 mmoles) d'une solution de méthylamine à 8N dans l'éthanol est ajouté puis le milieu, tube scellé, est chauffé à 60°C pour 3 heures d'agitation. Le milieu revenu à température ambiante est concentré sous vide et le résidu obtenu est chromatographié sur plaques préparatives de gel de silice en éluant avec un mélange 95/5/0,5 de dichlorométhane, de méthanol et d'ammoniaque à 30%. On obtient ainsi 0,073 g du produit attendu sous la forme d'une poudre blanche.
Point de fusion (°C) : 147-149°C
LC-MS : M+H = 434
RMN-¹H (DMSO) δ (ppm) : 8,85 (s, 1H); 7,35 (d, 2H); 6,90 (d, 2H) ; 6,80 (s, 1H) ; 5, 25 (s, 2H) ; 4, 75 (qt, 1H) ; 3, 35 (d large, 4H); 2,80 (s, 3H); 2,45 (m, 2H); 1,80 (m, 2H); 1,55 (dt, 4H).

### Exemple 4 (Composé N°4)

### 2-(4-Chloro-phénoxy)-7-aza-spiro[3.5]nonane-7-carboxylate de 3-carbamoyl-isoxazol-5-ylméthyle

### 4.1. 2-(4-Chloro-phénoxy)-7-aza-spiro[3.5]nonane-7-carboxylate de 3-carbamoyl-isoxazol-5-ylméthyl

Dans un tube scellé, 0,190 g (0,42 mmole) de 2-(4-chlorophénoxy)-7-aza-spiro[3.5]nonane-7-carboxylate de 3-éthoxycarbonyl-isoxazol-5-ylméthyle, obtenu à l'étape 3.1., est mis en solution dans 5 mL de méthanol. 0,91 mL (6,36 mmoles) d'une solution d'ammoniaque à 7N dans le méthanol est ajouté, et le milieu est chauffé à 50°C pendant 15 heures. Le milieu revenu à température ambiante est concentré sous vide et le résidu obtenu est chromatographié sur plaques préparatives de gel de silice en éluant avec un mélange 95/5/0,5 de dichlorométhane, de méthanol et d'ammoniaque à 30%. On obtient une huile qui cristallise dans le pentane. Le solide obtenu est filtré et séché sous vide à 60°C. On obtient 0,104 g du produit attendu sous la forme d'une poudre blanche.
Point de fusion (°C) : 70-72°C
LC-MS : M+H = 420
RMN-¹H (DMSO) δ (ppm) : 8,15 (s, 1H); 7,85 (s, 1H) ; 7,30 (d, 2H) ; 6,90 (d, 2H) ; 6,80 (s, 1H) ; 5,25 (s, 2H) ; 4,75 (qt, 1H); 3,35 (d large, 4H); 2,45 (t, 2H); 1,80 (t, 2H); 1,55 (d, 4H).

### Exemple 5 (Composé N°20)

### 2-(4'-Fluoro-biphényl-3-yloxy)-7-aza-spiro[3.5]nonane-7-carboxylate de 4-méthylcarbamoyl-thiazol-2-ylméthyle

### 5.1. 2-(4'-Fluoro-biphényl-3-yloxy)-7-aza-spiro[3.5]nonane

On procède suivant le mode opératoire décrit dans l'exemple 2, étapes 2.1. et 2.2.. A partir de 0,500 g (2,07 mmoles) de 2-hydroxy-7-aza-spiro[3.5]nonane-7-carboxylate de tert-butyle (WO2003084948), de 0,468 g (2,49 mmoles) de 4'-fluoro-biphényl-3-ol, de 0,440 g (2,53 mmoles) de diéthylazodicarboxylate, de 0,853 g (3,25 mmoles) de triphénylphosphine, et de 7,77 mL d'une solution d'acide chlorhydrique à 4N dans le dioxane, on obtient 0,645 g de produit attendu sous la forme d'une cire utilisée telle quelle dans l'étape suivante.

### 5.2. 2-Hydroxyméthyl-thiazole-4-carboxylate de méthyle

### 5.2.1 2-[(Acétyloxy)méthyl]-1,3-thiazole-4-carboxylate d'éthyle

2,7 g (10,80 mmoles) de 2-(bromométhyl) thiazole-4-carboxylate d'éthyle sont mis en solution dans 108 mL d'acétonitrile. 2,225 g (22,67 mmoles) d'acétate de potassium sont ajoutés et le milieu est agité à température ambiante pendant 14 heures.
On concentre sous pression réduite. Le résidu obtenu est repris dans une solution aqueuse saturée de chlorure de sodium et extrait deux fois au dichlorométhane. Les phases organiques groupées sont séchées sur sulfate de sodium, filtrées et concentrées à sec. On obtient 2,347 g de produit attendu sous la forme d'une cire.
RMN-¹H (CDCl₃) δ (ppm) : 8,15 (s, 1H) ; 5,35 (s, 2H) ; 4,35 (qd, 2 H) ; 2,10 (s, 3H) ; 1,35 (t, 3H).

### 5.2.2. 2-Hydroxyméthyl-thiazole-4-carboxylate de méthyle

2,347 g (10,24 mmoles) de 2-acétoxyméthyl-thiazole-4-carboxylate d'éthyle, obtenu à l'étape 5.2.1, sont mis en solution dans 100 mL d'un mélange 5/1 de dichlorométhane et de méthanol. 2,58 mL (11,26 mmoles) d'une solution de méthanolate de sodium à 4,37N dans le méthanol sont additionnés et le milieu est agité à température ambiante pendant deux heures avant d'être concentré sous pression réduite. Le résidu obtenu est repris par une solution aqueuse saturée de chlorure de sodium et extrait trois fois au dichlorométhane. Les phases organiques groupées sont lavées une fois avec une solution aqueuse saturée en chlorure de sodium, séchées sur sulfate de sodium, filtrées et concentrées à sec. Le résidu obtenu est purifié par chromatographie sur gel de silice en éluant avec un mélange 98/2/0,2 de dichlorométhane, de méthanol et d'ammoniaque à 30%. On obtient 0,92 g de produit attendu sous la forme d'une poudre blanche.
Point de fusion (°C): 158-160°C
RMN-¹H (CDCl₃) δ (ppm) : 8,10 (s, 1H) ; 4,95 (s, 2H) ; 3,90 (s, 3H); 2,50 (large s, 1H).

### 5.3. 2-(4'-Fluoro-biphényl-3-yloxy)-7-aza-spiro[3.5]nonane-7-carboxylate de 4-méthoxycarbonyl-thiazol-2-ylméthyl

On procède suivant le mode opératoire décrit dans l'exemple 2, étape 2.3. A partir de 0,19 g (0,61 mmole) de 2-(4'-fluoro-biphényl-3-yloxy)-7-aza-spiro[3.5]nonane, obtenu à l'étape 5.1., de 0,127 g (0,73 mmole) de 2-hydroxyméthyl-thiazole-4-carboxylate de méthyle, obtenu à l'étape 5.2.2., de 0,135 g (0,67 mmole) de para-nitrophényle chloroformiate et de 0,265 mL (1,53 mmoles) de N,N-diisopropyléthylamine, et après chromatographie sur gel de silice en éluant avec un mélange 99/1 de dichlorométhane et de méthanol, on obtient 0,161 g de produit attendu sous la forme d'une cire.
RMN-¹H (CDCl₃) δ (ppm): 8,10 (s, 1H) ; 7,45 (t, 2H) ; 7,25 (t, 1H); 7,05 (m, 3H); 6,90 (d, 1H); 6,70 (d, 1H); 5,35 (s, 2H); 4,70 (qt, 1H); 3,90 (s, 3H); 3,40 (m, 4H); 2,35 (m, 2H); 1,95 (m, 2H) ; 1,60 (m, 4H).

### 5.4. 2-(4'-Fluoro-biphényl-3-yloxy)-7-aza-spiro[3.5]nonane-7-carboxylate de 4-méthylcarbamoyl-thiazol-2-ylméthyle

Dans un tube scellé, 0,155 g (0,30 mmole) de 2-(4'-fluorobiphényl-3-yloxy)-7-aza-spiro[3.5]nonane-7-carboxylate de 4-méthoxycarbonyl-thiazol-2-ylméthyle, obtenu à l'étape 5.3., est mis en solution dans 6 mL d'éthanol. 1 mL (8,00 mmoles) d'une solution de méthylamine à 8N dans l'éthanol est ajouté, et le milieu est agité à température ambiante pendant 15 heures. Après concentration sous vide du milieu, le résidu obtenu est purifié par chromatographie sur gel de silice en éluant avec du dichlorométhane puis avec un mélange 99/1/0,1 de dichlorométhane, de méthanol et d'ammoniaque à 30%. On obtient une cire qui cristallise dans l'éther diisopropylique pour donner après filtration et séchage sous vide à 60°C, 0,087 g de produit attendu sous la forme d'une poudre blanche.
Point de fusion (°C) : 130-132°C
LC-MS : M+H = 510
RMN-¹H (DMSO) δ (ppm) : 8,35 (s large, 1H); 8,25 (s, 1H); 7,70 (t, 2H); 7,35 (t, 1H); 7,30 (t, 2H); 7,20 (d, 1H); 7,05 (s, 1H) ; 6, 85 (d, 1H) ; 5, 35 (s, 2H) ; 4,90 (qt, 1H) ; 3,40 (d large, 4H); 2,80 (s, 3H); 2,50 (m, 2H); 1,85 (m, 2H); 1,60 (dt, 4H).

### Exemple 6 (Composé N°17)

### 2-(4-Chloro-naphthalèn-1-yloxy)-7-aza-spiro[3.5]nonane-7-carboxylate de 3-méthylcarbamoyl-isoxazol-5-ylméthyle

### 6.1. 4-Nitro-phényl-carbonate de 3-méthylcarbamoyl-isoxazol-5-ylméthyle

A une solution de 2,00 g (12,81 mmoles) de 3-méthylcarbamoyl-isoxazol-5-ylméthanol (commercial), de 1,52 g (19,21 mmoles) de pyridine et de 0,157 g (1,28 mmoles) de N,N-diméthylaminopyridine dans 15 ml de dichlorométhane, refroidie à environ 0°C, sont ajoutés par petites portions 2,58 g (12,81 mmoles) de chloroformiate de 4-nitrophényle. Le milieu est maintenu sous agitation à 0°C pendant 1 heure puis à température ambiante pendant 1 heure. Le précipité formé est filtré puis rincé abondamment avec le diisopropyléther. Après séchage sous vide à environ 60°C, on obtient 2,6 g de produit pur sous forme de poudre blanche.
Point de fusion (°C): 166-168°C
RMN ¹H (CDCl₃) δ (ppm) : 8,40 (d, 2H) ; 7,50 (d, 2H) ; 7,0 (s, 1H) ; 6,90 (large s, 1H) ; 5,50 (s, 2H) ; 3,10 (d, 3H).

### 6.2. Chlorhydrate de 2-(4-chloro-naphthalèn-1-yloxy)-7-azaspiro[3.5]nonane

Sous atmosphère inerte, 1,00 g (4,14 mmoles) de 2-hydroxy-7-aza-spiro[3.5]nonane-7-carboxylate de tert-butyle (WO 2003084948), est mis en solution dans 41 mL de toluène. 1,250 g (4,77 mmoles) de triphénylphosphine et 0,888 g (4,97 mmoles) de 4-chloro- naphthalèn-1-ol (commercial) sont ajoutés. Le milieu est refroidi à 0°C et une solution de 0,794 g (4,56 mmoles) de diéthylazodicarboxylate dans 3 mL de toluène est additionnée. Le milieu est agité 14 heures à température ambiante puis concentré sous vide. Le résidu obtenu est repris avec une solution aqueuse de soude à 1N et extrait deux fois au dichlorométhane. Les phases organiques groupées sont lavées une fois avec une solution aqueuse saturée en chlorure de sodium, séchées sur sulfate de sodium, filtrées et concentrées sous vide. Le résidu brut obtenu est repris dans 20 mL de dichlorométhane et 10 mL (40 mmoles) d'une solution d'acide chlorhydrique à 4N dans le dioxane sont ajoutés lentement sous agitation. Après 3 heures d'agitation à température ambiante, le milieu est concentré sous vide et le résidu est repris dans une solution aqueuse d'acide chlorhydrique à 1N. La phase aqueuse est extraite deux fois à l'acétate d'éthyle puis lentement alcalinisée jusqu'à pH 10 par addition de soude à 35%. La phase aqueuse est extraite trois fois au dichlorométhane. Ces trois extraits organiques sont groupés, lavés une fois avec une solution aqueuse saturée en chlorure de sodium, séchés sur sulfate de sodium, filtrés et concentrés sous vide. On obtient le produit attendu sous la forme d'une cire qui est reprise avec 2 ml d'une solution d'acide chlorhydrique à 4N dans le dioxane. Le milieu est dilué avec de l'éther diéthylique et le précipité formé est filtré, rincé à l'éther diéthylique et séché sous vide. On obtient 1,10 g du chlorhydrate du produit attendu sous la forme d'une poudre blanche.
Point de fusion (°C): 272-274°C
LC-MS : M+H = 302
RMN-¹H (DMSO) δ (ppm) : 8,75 (s large, 2H); 8,25 (d, 1H); 8,15 (d, 1H); 7,75 (t, 1H); 7,65 (t, 1H), 7,60 (d, 1H); 6,80 (d, 1H); 4,95 (qt, 1H); 3,00 (d large, 4H); 2,60 (m, 2H); 2,05(m, 2H); 1,85 (dt, 4H).

### 6.3. 2-(4-Chloro-naphthalèn-1-yloxy)-7-aza-spiro[3.5]nonane-7-carboxylate de 3-méthylcarbamoyl-isoxazol-5-ylméthyle

Dans un tube réacteur, un mélange de 0,300 g (0,89 mmole) de chlorhydrate de 2-(4-chloro-naphthalèn-1-yloxy)-7-azaspiro[3.5]nonane, obtenu à l'étape 6.2., de 0,342 g (1,06 mmoles) de 4-nitro-phényl-carbonate de 3-méthylcarbamoyl-isoxazol-5-ylméthyle, obtenu à l'étape 6.1., de 0,54 mL (3,10 mmoles) de N,N-diisopropyléthylamine et de 0,011 g (0,09 mmole) de N,N- diméthylaminopyridine , est mis en solution dans 5 mL de 1,2- dichloroéthane. Le milieu est ensuite porté à 70°C pendant 14 heures. Après retour à température ambiante, le milieu est dilué avec une solution aqueuse de soude à 1N, et extrait deux fois avec du dichlorométhane. Les phases organiques réunies sont ensuite successivement lavées deux fois par une solution aqueuse de soude à 1N, une fois par une solution aqueuse saturée de chlorure d'ammonium, et une fois par une solution aqueuse saturée de chlorure de sodium. Après séchage sur sulfate de sodium, filtration et évaporation à sec, le résidu obtenu est purifié par chromatographie sur gel de silice en éluant avec du dichlorométhane puis avec un mélange 99/1/0,1 de dichlorométhane, de méthanol et d'ammoniaque à 30%. On obtient 0,363 g de produit attendu sous la forme d'une poudre blanche.
Point de fusion (°C): 62-64°C
LC-MS : M+H = 484
RMN-¹H (DMSO) δ (ppm) : 8,70 (s, 1H); 8,25 (d, 1H); 8,15 (d, 1H); 7,75 (t, 1H); 7,65 (t, 1H), 7,60 (d, 1H); 6,80 (d, 2H); 5,25 (s, 2H); 4,95 (qt, 1H); 3,40 (d large, 4H); 2,80 (s, 3H); 2,55 (m, 2H); 1,95 (m, 2H); 1,60 (dt, 4H).

### Exemple 7 (Composé N°30)

### 2-(4-Chloro-naphthalèn-1-yloxy)-6-aza-spiro[3.4]octane-6-carboxylate de 3-carbamoyl-isoxazol-5-ylméthyle

### 7.1. 2-Hydroxy-6-aza-spiro[3.4]octane-6-carboxylate de tert-butyle

A une solution de 3,54 g (15,71 mmoles) de 2-oxo-6-azaspiro[3.4]octane-6-carboxylate de tert-butyle (WO9806720) dilué dans 40 mL de méthanol, est ajouté 0,89g (23,57 mmoles) de borohydrure de sodium par portions à 0 °C. Le mélange réactionnel est agité à température ambiante pendant 1 heure et 30 minutes. Après évaporation du solvant, on ajoute de l'eau au milieu réactionnel, on sépare la phase aqueuse, on l'extrait plusieurs fois avec de l'éther diéthylique, on lave les phases organiques réunies avec une solution aqueuse saturée en chlorure de sodium, on les sèche sur sulfate de sodium et on concentre le filtrat sous pression réduite. Après évaporation du solvant, on obtient 3,10 g de produit sous forme d'une huile marron utilisée telle quelle dans l'étape suivante.
RMN ¹H (DMSO) δ (ppm) : 4,7 (t, 1H) ; 4,1 (m, 1H) ; 3,2 (m, 4H), ; 2,2 (m, 2H) ; 1,8 (m, 4H) ; 1,4 (s, 9H).

### 7.2. 2-(4-Chloro-naphthalèn-1-yloxy)-6-aza-spiro[3.4]octane-6-carboxylate de tert-butyle

On procède suivant le mode opératoire décrit dans l'exemple 2, étape 2.1.. A partir de 3,50 g (15,40 mmoles) de 2-hydroxy-6-aza-spiro[3.4]octane-6-carboxylate de tert-butyle, obtenu à l'étape 7.1., de 3,30 g (18,48 mmoles) de 4-chloronaphthalèn-1-ol (commercial), de 3,08 g (17,71 mmoles) de diéthylazodicarboxylate et de 4,846 g (18,48 mmoles) de triphénylphosphine, et après chromatographie sur gel de silice en éluant avec un mélange 70/30, 60/40 puis 50/50 de cyclohexane et d'acétate d'éthyle, on obtient 6,14 g de produit attendu sous la forme d'une huile, utilisée telle quelle dans l'étape suivante.

### 7.3 Trifluoroacétate de 2-(4-chloro-naphthalèn-1-yloxy)-6-aza-spiro[3.4]octane

6,14 g de 2-(4-chloro-naphthalèn-1-yloxy)-6-azaspiro[3.4]octane-6-carboxylate de tert-butyle, obtenu à l'étape 7.2., sont mis en solution dans 100 ml de dichlorométhane. Le milieu est refroidi à 0°C puis 20 ml d'acide trifluoroacétique sont lentement additionnés. Après 2 heures d'agitation à température ambiante, le milieu est dilué avec 100 mL de toluène et concentré sous vide. Le résidu obtenu est repris dans l'éther diéthylique pour donner une poudre qui est filtrée, rincée à l'éther diéthylique et séchée sous vide. On obtient 4,19 g du produit attendu sous la forme d'une poudre rose.
Point de fusion (°C) : 129-131°C
RMN-¹H (DMSO) δ (ppm) : 8,85 (s large, 2H) ; 8,25 (d, 1H) ; 8, 15 (d, 1H) ; 7,75 (t, 1H) ; 7,65 (t, 1H); 7,60 (d, 1H); 6,85 (dd, 1H); 5,00 (qt, 1H); 3,25 (m, 4H); 2,75 (m, 1H); 2,65 (m, 1H); 2,30 (m, 2H); 2,10 (m, 2H).

### 7.4. 4-Nitro-phényl-carbonate de 3-carbamoyl-isoxazol-5-ylméthyle

A une solution de 2,0 g (14,07 mmoles) de 3-carbamoyl-isoxazol-5-ylméthanol (commercial), de 1,71 ml (21,11 mmoles) de pyridine et de 0,17 g (1,41 mmoles) de N,N-diméthylaminopyridine dans 15 ml de dichlorométhane, refroidie à environ 0°C, sont ajoutés par petites portions 2,84 g (14,07 mmoles) de chloroformiate de 4-nitrophényle. Le milieu est maintenu sous agitation à 0°C pendant 1 heure puis à température ambiante pendant 1 heure. Le précipité formé est filtré puis rincé abondamment avec le diisopropyléther. Après séchage sous vide à environ 60°C, on obtient 3,12 g de produit attendu sous la forme d'un solide blanc utilisé tel quel dans l'étape suivante.
Point de fusion (°C): 143-145°C
RMN ¹H (DMSO) δ (ppm) : 8,40(d, 2H) ; 8,25 (large s, 1H) ; 7,90 (large s, 1H) ; 7,65 (d, 2H) ; 7,0 (s, 1H) ; 5,50 (s, 2H).

### 7.5. 2-(4-Chloro-naphthalèn-1-yloxy)-6-aza-spiro[3.4]octane-6-carboxylate de 3-carbamoyl-isoxazol-5-ylméthyle

Dans un tube réacteur, un mélange de 1,000 g (2,49 mmoles) de trifluoroacétate de 2-(4-chloro-naphthalèn-1-yloxy)-6-aza-spiro[3.4]octane, obtenu à l'étape 7.3., de 0,841 g (2,74 mmoles) de 4-nitro-phényl-carbonate de 3-carbamoyl-isoxazol-5-ylméthyle, obtenu à l'étape 7.4., de 1,30 mL (7,47 mmoles) de N,N-diisopropyléthylamine et 0,032 g (0,25 mmole) de N,N-diméthylaminopyridine, est mis en solution dans 8 mL de 1,2-dichloroéthane. Le milieu, tube scellé, est porté à 70°C pour 14 heures d'agitation. Après retour à température ambiante, le milieu est dilué avec une solution aqueuse de soude à 1N et extrait deux fois avec du dichlorométhane. Les phases organiques réunies sont ensuite successivement lavées deux fois par une solution aqueuse de soude à 1N, une fois par une solution aqueuse saturée de chlorure d'ammonium, et une fois par une solution aqueuse saturée de chlorure de sodium. Après séchage sur sulfate de sodium, filtration et évaporation à sec, le résidu est purifié par chromatographie sur gel de silice en éluant avec un mélange 99/1/0,1 puis 98/2/0,2 de dichlorométhane, de méthanol et d'ammoniaque à 30%. On obtient 1,15 g de produit attendu sous la forme d'une poudre blanche.
Point de fusion (°C) : 149-150°C
LC-MS : M+H = 456
RMN-¹H (DMSO) δ (ppm) : 8,25 (d, 1H); 8,15 (d, 2H); 7,85 (s large, 1H); 7,75 (t, 1H); 7,65 (t, 1H); 7,55 (d, 1H); 6,85 (d, 1H); 6,80 (s, 1H); 5,25 (d, 2H); 5,00 (m, 1H); 3,40 (m, 4H) ; 2,65 (m, 2H) ; 2,20 (m, 2H) ; 2,00 (m, 2H).

### Exemple 8 (Composés N°10 et 11)

### 2-(3-Trifluorométhyl-phénoxy)-6-aza-spiro[3.4]octane-6-carboxylate de 3-carbamoyl-isoxazol-5-ylméthyle (isomères I et II)

### 8.1. 2-(3-Trifluorométhyl-phénoxy)-6-aza-spiro[3.4]octane-6-carboxylate de tert-butyle (isomères 1a et 1b)

0,910 g (4,00 mmoles) de 2-hydroxy-6-aza-spiro[3.4]octane-6-carboxylate de tert-butyle, obtenu à l'étape 7.1., est mis en solution dans 14 ml de diméthylformamide. 0,240 g (6,01 mmoles) d'hydrure de sodium est additionné par portions puis 0,821 g (5,00 mmoles) de 1-fluoro-3-trifluorométhyl-benzène (commercial) est ajouté au milieu. Après 14 heures de chauffage à 90°C, le milieu est laissé revenir à température ambiante puis dilué dans de l'eau et de l'acétate d'éthyle. Après décantation et séparation, la phase aqueuse est extraite une deuxième fois à l'acétate d'éthyle puis les phases organiques réunies sont lavées deux fois avec de l'eau et une fois avec une solution aqueuse saturée en chlorure de sodium, séchées sur sulfate de sodium et concentrées à sec. On obtient 1,28 g des isomères 1a et 1b attendus sous la forme d'une huile.
0,130 g (0,35 mmole) du mélange des isomères, est séparé par chromatographie sur plaques préparatives de gel de silice en éluant avec un mélange 70/30 de cyclohexane et d'acétate d'éthyle.
On obtient ainsi 0,042 g de l'isomère 1a (R_{f} = 0,55) et 0,045 g de l'isomère 1b (R_{f} = 0,65) sous forme d'huiles incolores.

### Isomère 1a

### R_{f} = 0,55 (acétate d'éthyle / cyclohexane : 70 / 30)

RMN ¹H (DMSO) δ (ppm) : 7,55 (t, 1H) ; 7,30 (d, 1H); 7,20 (d, 1H) ; 7,15 (s, 1H) ; 4,90 (qt, 1H) ; 3,35 (d, 2H); 3,25 (m, 2H); 2,55 (m, 2H); 2,05 (m, 2H); 1,90 (m, 2H) ; 1,45 (s, 9H).

### Isomère 1b

### R_{f} = 0,65 (acétate d'éthyle / cyclohexane : 70 / 30)

RMN ¹H (DMSO) δ (ppm): 7,55 (t, 1H) ; 7,30 (d, 1H); 7,20 (d, 1H); 7,15 (s, 1H); 4,85 (qt, 1H); 3,35-3,20 (m, 4H); 2,50 (m, 2H); 2,10 (m, 2H); 1,95 (m, 2H) ; 1,40 (s, 9H).

### 8.2. Chlorhydrate de 2-(3-trifluorométhyl-phénoxy)-6-azaspiro[3.4]octane

### 8.2.a. Chlorhydrate de 2-(3-trifluorométhyl-phénoxy)-6-azaspiro[3.4]octane (obtenu à partir de l'isomère 1a)

0,042 g (0,11 mmole) de 2-(3- trifluorométhyl-phénoxy)-6-aza-spiro[3.4]octane-6-carboxylate de tert-butyle, isomère 1a, obtenu à l'étape 8.1., est repris dans 3 mL de dioxane et 0,43 mL (1,71 mmoles) d'une solution d'acide chlorhydrique à 4N dans le dioxane est ajouté lentement sous agitation. Après 14 heures d'agitation à température ambiante, le milieu est concentré à sec sous pression réduite. On obtient 0,035 g du chlorhydrate du produit attendu sous la forme d'une huile jaune.

### 8.2.b Chlorhydrate de 2-(3-trifluorométhyl-phénoxy)-6-azaspiro[3.4]octane (obtenu à partir de l'isomère 1b)

0,045 g (0,12 mmole) de 2-(3-trifluorométhyl-phénoxy)-6-azaspiro[3.4]octane-6-carboxylate de tert-butyle, isomère 1b, obtenu à l'étape 8.1., est repris dans 3 mL de dioxane et 0,45 mL (1,82 mmoles) d'une solution d'acide chlorhydrique à 4N dans le dioxane est ajouté lentement sous agitation. Après 14 heures d'agitation à température ambiante, le milieu est concentré à sec sous pression réduite. On obtient 0,037 g du chlorhydrate du produit attendu sous la forme d'une huile jaune.

### 8.3. 2-(3-Trifluorométhyl-phénoxy)-6-aza-spiro[3.4]octane-6-carboxylate de 3-carbamoyl-isoxazol-5-ylméthyle

### 8.3.a. 2-(3-Trifluorométhyl-phénoxy)-6-aza-spiro[3.4]octane-6-carboxylate de 3-carbamoyl-isoxazol-5-ylméthyle (isomère I) (composé n°10).

On procède suivant le mode opératoire décrit dans l'exemple 6, étape 6.3.. A partir de 0,035 g (0,11 mmole) de chlorhydrate de 2-(3-trifluorométhyl-phénoxy)-6-azaspiro[3.4]octane, obtenu à l'étape 8.2.a., de 0,042 g (0,14 mmole) de 4-nitro-phényl-carbonate de 3-carbamoyl-isoxazol-5-ylméthyle, obtenu à l'étape 7.4., de 0,060 mL (0,34 mmole) de N,N-diisopropyléthylamine, et de 0,007 g (0,06 mmole) de N,N-diméthylaminopyridine, et après purification par chromatographie sur plaques préparatives de gel de silice en éluant avec un mélange 90/10/1 de dichlorométhane, de méthanol et d'ammoniaque à 30%, on obtient une huile incolore qui cristallise dans le pentane. Après filtration et séchage sous vide à 60°C, on obtient 0,050 g du produit attendu sous la forme d'une poudre blanche.
LC-MS : M+H = 440
Point de fusion (°C):97-99°C
RMN-¹H (DMSO) δ (ppm) : 8,15 (s, 1H); 7,85 (s, 1H); 7,55 (t, 1H); 7,30 (d, 1H); 7,20 (d, 1H), 7,15 (s, 1H); 6,85 (d, 1H); 5,25 (s, 2H); 4,90 (qt, 1H); 3,45 (s, 2H); 3,30 (m, 2H); 2,55 (m, 2H); 2,05 (m, 2H); 1,95 (m, 2H).

### 8.3.b 2-(3-Trifluorométhyl-phénoxy)-6-aza-spiro[3.4]octane-6-carboxylate de 3-carbamoyl-isoxazol-5-ylméthyle (isomère II) (composé n°11).

On procède suivant le mode opératoire décrit dans l'exemple 6, étape 6.3. A partir de 0,037 g (0,12 mmole) de chlorhydrate de 2-(3-trifluorométhyl-phénoxy)-6-azaspiro[3.4]octane, obtenu à l'étape 8.2.b., de 0,042 g (0,14 mmole) de 4-nitro-phényl-carbonate de 3-carbamoyl-isoxazol-5-ylméthyle, obtenu à l'étape 7.4., de 0,060 mL (0,36 mmole) de N,N-diisopropyléthylamine, et de 0,007 g (0,06 mmole) de N,N-diméthylaminopyridine, et après purification par chromatographie sur plaques préparatives de gel de silice en éluant avec un mélange 90/10/1 de dichlorométhane, de méthanol et d'ammoniaque à 30%, on obtient une huile incolore qui cristallise dans le pentane. Après filtration du solide et séchage sous vide à 60°C, on obtient 0,029 g du produit attendu sous la forme d'une cire.
LC-MS: M+H = 440
RMN-¹H (DMSO) δ (ppm) : 8,10 (s, 1H); 7,85 (s, 1H); 7,55 (t, 1H); 7,30 (d, 1H); 7,20 (d, 1H), 7,15 (s, 1H); 6,80 (s, 1H); 5,25 (s, 2H); 4,85 (qt, 1H); 3,45-3,25 (m, 4H); 2,50 (m, 2H); 2,10 (m, 2H); 1,95 (m, 2H).

### Exemple 9 (Composés N°32 et 33)

### 2-(4'-Fluoro-biphényl-4-yloxy)-6-aza-spiro[3.4]octane-6-carboxylate de 3-méthylcarbamoyl-isoxazol-5-ylméthyle (isomères I et II)

### 9.1 2-(Toluène-4-sulfonyloxy)-6-aza-spiro[3.4]octane-6-carboxylate de tert-butyle (isomères 2a et 2b)

10,00 g (43,99 mmoles) de 2-hydroxy-6-aza-spiro[3.4]octane-6-carboxylate de tert-butyle, obtenu à l'étape 7.1., et 9,15 mL (65,98 mmoles) de triéthylamine, sont dissous dans 400 mL de dichlorométhane et 13,07 g (65,98 mmoles) de chlorure de tosyle sont ajoutés. Après 14 heures d'agitation à température ambiante, le milieu est extrait une fois avec une solution aqueuse saturée en chlorure d'ammonium puis une fois avec une solution aqueuse saturée en chlorure de sodium, séché sur sulfate de sodium, filtré et concentré sous pression réduite. Le résidu obtenu est purifié par chromatographie sur gel de silice en éluant avec du cyclohexane puis avec un mélange 95/5 de cyclohexane et d'acétate d'éthyle. On obtient 11,9 g de produit attendu sous la forme d'une huile.
LC-MS : M+H = 382
RMN-¹H (DMSO) δ (ppm) : 7,80 (d, 2H) ; 7,50 (d, 2H) ; 4,90 (m, 1H); 3,15 (m, 4H); 2,45 (s, 3H); 2,20 (m, 2H); 2,10 (m, 2H); 1,80 (t, 2H); 1,40 (s, 9H).

11,5 g (3,01 mmoles) de 2-(toluène-4-sulfonyloxy)-6-azaspiro[3.4]octane-6-carboxylate de tert-butyle, obtenu à l'étape 9.1., sont séparés par chromatographie chirale préparative (Macherey-Nagel Nucléosil 50-10 / 50X220 mm) en éluant avec un mélange 13/87 d'acétate d'éthyle et de cyclohexane, pour donner 5,15 g d'isomère 2a et 4,69 g d'isomère 2b sous la forme de poudres blanches.

### Isomère 2a

Tr = 33 min
Point de fusion (°C): 83,7°C
LC-MS : M+H = 382
RMN-¹H (DMSO) δ (ppm) : 7,80 (d, 2H); 7,50 (d, 2H); 4,90 (qt, 1H); 3,15 (m, 4H); 2,45 (s, 3H); 2,25 (t, 2H); 2,05 (t, 2H); 1,80 (m, 2H); 1,40 (s, 9H).

### Isomère 2b

Tr = 43 min
Point de fusion (°C): 94,9°C
LC-MS : M+H = 382
RMN ¹H (DMSO) δ (ppm) : 7,80 (d, 2H); 7,50 (d, 2H); 4,90 (qt, 1H); 3,20 (m, 4H); 2,45 (s, 3H); 2,20 (t, 2H); 2,10 (t, 2H); 1,80 (m, 2H); 1,40 (s, 9H).

### 9.2. 2-(4'-Fluoro-biphényl-4-yloxy)-6-aza-spiro[3.4]octane-6-carboxylate de tert-butyle

### 9.2.a. 2-(4'-Fluoro-biphényl-4-yloxy)-6-azaspiro[3.4]octane-6-carboxylate de tert-butyle (obtenu à partir de l'isomère 2a)

0,542 g (2,88 mmoles) de 4'- fluoro-biphényl-4-ol, 0,362 (2,62 mmoles) de carbonate de potassium et 0,693 g (2,62 mmoles) de 18-crown-6, sont mis en solution dans 26 mL de N,N-diméthylformamide. Le milieu est porté à 80°C sous agitation puis 1,00 g (2,62 mmoles) du 2-(toluène-4-sulfonyloxy)-6-aza-spiro[3.4]octane-6-carboxylate de tert-butyle, isomère 2a, obtenu à l'étape 9.1., en solution dans 4 mL de N,N-diméthylformamide, est additionné goutte à goutte. Après 14 heures d'agitation à 80°C, le milieu est laissé revenir à température ambiante puis concentré à sec sous pression réduite. Le résidu est repris dans le dichlorométhane et une solution aqueuse de soude à 1N. Après décantation, la phase aqueuse est séparée et ré-extraite deux fois au dichlorométhane. Les phases organiques groupées sont lavées avec une solution aqueuse saturée en chlorure de sodium, séchées sur sulfate de sodium et concentrées sous pression réduite. Le résidu obtenu est purifié par chromatographie sur gel de silice en éluant avec un mélange 90/10 de cyclohexane et d'acétate d'éthyle. On obtient 0,80 g de produit attendu sous la forme d'une poudre.
Point de fusion (°C) : 132-133°C
LC-MS : M+H = 398
RMN-¹H (DMSO) δ (ppm) : 7,65 (t, 2H); 7,55 (d, 2H); 7,25 (t, 2H), 6,95 (d, 2H); 4,80 (qt, 1H); 3,25 (m, 4H); 2,50 (m, 2H); 2,15 (m, 2H); 1,95 (m, 2H) ; 1,40 (s, 9H).

### 9.2.b. 2-(4'-Fluoro-biphényl-4-yloxy)-6-azaspiro[3.4]octane-6-carboxylate de tert-butyle (obtenu à partir de l'isomère 2b)

On procède suivant le mode opératoire décrit dans l'exemple 9, étape 9.2.a.. A partir de 1,2 g (3,15 mmoles) de 2-(toluène-4-sulfonyloxy)-6-aza-spiro[3.4]octane-6-carboxylate de tert-butyle, isomère 2b., obtenu à l'étape 9.1., de 0,622 g (3,30 mmoles) de 4'-fluoro-biphenyl-4-ol, de 0,435 g (3,15 mmoles) de carbonate de potassium, et de 0,831 g (3,15 mmoles) de 18-crown-6 , et après purification par chromatographie sur gel de silice en éluant avec un mélange 90/10 de cyclohexane et d'acétate d'éthyle, on obtient 0,860 g de produit attendu sous la forme d'une cire utilisée telle quelle dans l'étape suivante.
RMN-¹H (DMSO) δ (ppm) : 7,65 (t, 2H); 7,55 (d, 2H); 7,25 (t, 2H), 6,95 (d, 2H) ; 4,85 (qt, 1H); 3,30 (m, 2H) ; 3,25 (m, 2H); 2,55 (m, 2H); 2,05 (m, 2H); 1,90 (m, 2H) ; 1,40 (s, 9H).

### 9.3. Chlorhydrate de 2-(4'- fluoro-biphényl-4-yloxy)-6-aza-spiro[3.4]octane

### 9.3.a. Chlorhydrate de 2-(4'-fluoro-biphényl-4-yloxy)-6-azaspiro[3.4]octane (obtenu à partir de l'isomère 2a)

1,000 g (2,52 mmoles) de 2-(4'-fluoro-biphényl-4-yloxy)-6-aza-spiro[3.4]octane-6-carboxylate de tert-butyle, obtenu à l'étape 9.2.a. à partir de l'isomère 2a, est mis en solution dans 8 mL de dioxane et 6,29 mL (25,16 mmoles) d'une solution d'acide chlorhydrique à 4 N dans le dioxane sont lentement ajoutés au milieu. Après 4 heures d'agitation à température ambiante, le milieu est concentré à sec sous pression réduite et le résidu obtenu repris dans l'éther diéthylique. Après 2 heures d'agitation, le solide en suspension est filtré, abondamment rincé à l'éther diéthylique et séché sous vide. On obtient 0,690 g de produit attendu sous la forme d'une poudre blanche.
Point de fusion (°C) : 227-229°C
LC-MS : M+H = 298
RMN-¹H (DMSO) δ (ppm) : 9,10 (s large, 2H) ; 7,65 (t, 2H) ; 7,60 (d, 2H); 7,25 (t, 2H), 6,95 (d, 2H); 4,80 (qt, 1H); 3,20 (m, 4H); 2,55 (m, 2H); 2,20 (m, 2H); 2,05 (m, 2H).

### 9.3.b. Chlorhydrate de 2-(4'-fluoro-biphényl-4-yloxy)-6-azaspiro[3.4]octane (obtenu à partir de l'isomère 2b)

On procède suivant le mode opératoire décrit dans l'exemple 9, étape 9.3.a. A partir de 0,860 g (2,16 mmoles) de 2-(4'-fluoro-biphényl-4-yloxy)-6-aza-spiro[3.4]octane-6-carboxylate de tert-butyle, obtenu à l'étape 9.2.b. à partir de l'isomère 2b, et de 15 mL (60,00 mmoles) d'une solution d'acide chlorhydrique à 4N dans le dioxane, on obtient 0,545 g de produit attendu sous la forme d'une poudre blanche.
Point de fusion (°C) : 233-235°C
LC-MS : M+H = 298
RMN-¹H (DMSO) δ (ppm) : 9,15 (s large, 2H) ; 7,65 (t, 2H) ; 7,60 (d, 2H); 7,25 (t, 2H), 6,95 (d, 2H); 4,80 (qt, 1H); 3,25 (s, 2H); 3,15 (m, 2H) ; 2,65 (t, 2H); 2,15 (t, 2H); 2,00 (t, 2H).

### 9.4. 2-(4'-Fluoro-biphényl-4-yloxy)-6-aza-spiro[3.4]octane-6-carboxylate de 3-méthylcarbamoyl-isoxazol-5-ylméthyle

### 9.4.a. 2-(4'-Fluoro-biphényl- 4-yloxy)-6-azaspiro[3.4]octane-6-carboxylate de 3-méthylcarbamoylisoxazol-5-ylméthyle (obtenu à partir de l'isomère 2a) (composé 32) (isomère I)

Dans un tube réacteur, un mélange de 0, 690 g (2,07 mmoles) de chlorhydrate de 2-(4'-Fluoro-biphényl-4-yloxy)-6-azaspiro[3.4]octane, obtenu à l'étape 9.3.a., de 0,730 g (2,27 mmoles) de 4-nitro-phényl-carbonate de 3-méthylcarbamoyl-isoxazol-5-ylméthyle, obtenu à l'étape 6.1, de 1,08 mL (6,20 mmoles) de N,N-diisopropyléthylamine et de 0,126 g (1,03 mmoles) de N,N-diméthylaminopyridine , est mis en solution dans 10 mL de 1,2-dichloroéthane. Le milieu, tube scellé, est porté à 70°C pour 14 heures d'agitation. Après retour à température ambiante, le milieu est dilué avec une solution aqueuse de soude à 1N, et extrait deux fois avec du dichlorométhane. Les phases organiques réunies sont ensuite successivement lavées deux fois par une solution aqueuse de soude à 1N, une fois par une solution aqueuse saturée de chlorure d'ammonium, et une fois par une solution aqueuse saturée de chlorure de sodium. Après séchage sur sulfate de sodium, filtration et évaporation sous pression réduite, le résidu est purifié par chromatographie sur gel de silice en éluant avec un mélange 98/2/0,2 de dichlorométhane, de méthanol et d'ammoniaque à 30%. On obtient 0,875 g de produit attendu sous la forme d'une poudre blanche.
Point de fusion (°C) : 149-151°C
LC-MS : M+H = 480
RMN-¹H (DMSO) δ (ppm) : 8,70 (s, 1H); 7,65 (t, 2H); 7,55 (d, 2H); 7,25 (t, 2H), 6,95 (d, 2H); 6,80 (s, 1H); 5,25 (s, 2H); 4,80 (qt, 1H); 3,35 (m, 4H); 2,80 (s, 3H); 2,50 (t, 2H); 2,15 (t, 2H); 1,95 (m, 2H).

### 9.4.b. 2-(4'-Fluoro-biphényl-4-yloxy)-6-azaspiro[3.4]octane-6-carboxylate de 3-méthylcarbamoyl-isoxazol-5-ylméthyle (obtenu à partir de l'isomère 2b) (composé n°33) (isomère II)

On procède suivant le mode opératoire décrit dans l'exemple 9, étape 9.4.a.. A partir de 0,53 g (1,59 mmoles) de chlorhydrate de 2-(4'-fluoro-biphényl-4-yloxy)-6-azaspiro[3.4]octane, obtenu à l'étape 9.3.b., de 0,561 g (1,75 mmoles) de 4-nitro-phényl-carbonate de 3-méthylcarbamoyl-isoxazol-5-ylméthyle, obtenu à l'étape 6.1, de 0,83 mL (4,76 mmoles) de N,N-diisopropyléthylamine et de 0,097 g (1,03 mmoles) de N,N-diméthylaminopyridine, et après purification par chromatographie sur gel de silice en éluant avec un mélange 98/2/0,2 de dichlorométhane, de méthanol et d'ammoniaque, on obtient 0,638 g de produit attendu sous la forme d'une poudre blanche.
Point de fusion (°C) : 150-152°C
LC-MS : M+H = 480
RMN-¹H (DMSO) δ (ppm) : 8,70 (s large, 1H); 7,65 (t, 2H); 7,55 (d, 2H); 7,25 (t, 2H), 6,95 (d, 2H); 6,80 (d, 1H); 5,25 (s, 2H); 4,85 (qt, 1H); 3,45 (m, 2H); 3,35 (m, 2H) ; 2,80 (s, 3H); 2,50 (m, 2H); 2,10 (t, 2H); 1,95 (m, 2H).

### Exemple 10 (Composé N°24)

### 6-(4-Chloro-3-fluoro-phénoxy)-2-aza-spiro[3.3]heptane-2-carboxylate de 3-carbamoyl-isoxazol-5-ylméthyle

### 10.1 5,5-Dichloro-6-oxo-2-aza-spiro[3.3]heptane-2-carboxylate de tert-butyle

Sous atmosphère inerte, 7,728 g (118,19 mmoles) de zinc sous forme de nanopoudre sont mis en suspension dans 100 ml d'éther diéthylique. 5,00 g (29,55 mmoles) de 3-méthylène-azétidine-1-carboxylate de tert-butyle (WO 2008124085) sont ajoutés puis le milieu est refroidi à 10°C. Une solution de 6,60 mL (59,09 mmoles) de chlorure de trichloroacétyle dans 30 mL de 1,2-diméthoxyéthane est additionnée goutte à goutte en maintenant la température du milieu réactionnel comprise entre 26 et 30°C. Après 14 heures d'agitation à température ambiante, on filtre le milieu sur célite, on rince abondamment la célite à l'éther diéthylique et le filtrat est partiellement concentré sous pression réduite. Le brut réactionnel ainsi obtenu est utilisé tel quel dans l'étape suivante.

### 10.2 6-Oxo-2-aza-spiro[3.3]heptane-2-carboxylate de tert-butyle

9,66 g (147,73 mmoles) de poudre de Zn sont mis en suspension dans 150 ml d'acide acétique glacial. Le milieu est refroidi à 0°C et la solution brute de 5,5-dichloro-6-oxo-2-aza-spiro[3.3]heptane-2-carboxylate de tert-butyle dans 30 mL de dioxane, obtenue à l'étape 10.1. est additionnée goutte à goutte. Après 16 heures d'agitation à température ambiante, le milieu est versé lentement par portions sur une solution aqueuse saturée en carbonate de sodium. Après réalcalinisation au carbonate de sodium, le milieu est agité une heure puis filtré sur fritté et le filtrat est extrait trois fois à l'acétate d'éthyle. Les phases organiques réunies sont lavées une fois avec une solution aqueuse saturée en chlorure de sodium, séchées sur sulfate de sodium, filtrées et concentrées sous vide. Le résidu est purifié par chromatographie sur gel de silice en éluant avec un mélange 90/10 puis 80/20 et 60/40 de cyclohexane et d'acétate d'éthyle. On obtient 2,4 g de produit attendu sous la forme d'une gomme brune.
RMN-¹H (DMSO) δ (ppm) : 4,05 (s, 4H); 3,30 (s, 4H); 1,40 (s, 9H).

### 10.3. 6-Hydroxy-2-aza-spiro[3.3]heptane-2-carboxylate de tert-butyle

A une solution de 2,30 g (10,89 mmoles) de 6-oxo-2-azaspiro[3.3]heptane-2-carboxylate de tert-butyle, obtenu à l'étape 10.2., dans 55 mL de méthanol, est ajouté 0,494 g (13,06 mmoles) de borohydrure de sodium par portions à 0°C. Le mélange réactionnel est agité à température ambiante pendant 1 heure. Après évaporation du solvant, on ajoute de l'eau au milieu réactionnel, on sépare la phase aqueuse, on l'extrait plusieurs fois avec du dichlorométhane, on lave les phases organiques réunies avec une solution aqueuse saturée en chlorure de sodium, on les sèche sur sulfate de sodium et on concentre le filtrat sous pression réduite. Après cristallisation du résidu dans l'éther diisopropylique, filtration du solide obtenu et séchage sous vide à 60°C, on obtient 2,24 g de produit sous forme d'une poudre beige.
RMN-¹H (DMSO) δ (ppm): 5,00 (d, 1H); 3,95 (hex, 1H); 3,75 (d, 4H); 2,40 (m, 2H); 1,95 (m, 2H); 1,40 (s, 9H).

### 10.4. 6-(4-Chloro-3-fluoro-phénoxy)-2-aza-spiro[3.3]heptane-2-carboxylate de tert-butyle

On procède suivant le mode opératoire décrit dans l'exemple 2, étape 2.1.. A partir de 1,50 g (7,03 mmoles) de 6-hydroxy-2-aza-spiro[3.3]heptane-2-carboxylate de tert-butyle, obtenu à l'étape 10.3., de 1,237 g (8,44 mmoles) de 4-chloro-3-fluoro-phénol, de 1,409 g (8,09 mmoles) de diéthylazodicarboxylate et de 2,121 g (8,09 mmoles) de triphénylphosphine, et après chromatographie sur gel de silice en éluant avec un mélange 95/5 puis 90/10 de cyclohexane et d'acétate d'éthyle, on obtient 1,73 g de produit attendu sous la forme d'une poudre beige.
Point de fusion (°C): 110- 112°C
LC-MS : M+H = 342
RMN-¹H (DMSO) δ (ppm) : 7,45 (t, 1H) ; 6,95 (d, 1H) ; 6,75 (d, 1H); 4,65 (qt, 1H); 3,90 (d large, 4H); 2,75 (m, 2H); 2,20 (m, 2H); 1,40 (s, 9H).

### 10.5. Trifluoroacétate de 6-(4-chloro-3-fluoro-phénoxy)-2-aza-spiro[3.3]heptane

1,70 g (4,97 mmoles) de 6-(4-chloro-3-fluoro-phénoxy)-2-azaspiro[3.3]heptane-2-carboxylate de tert-butyle, obtenu à l'étape 10.4., sont mis en solution dans 40 ml de dichlorométhane. Le milieu est refroidi dans un bain de glace puis 8 mL (105,24 mmoles) d'acide trifluoroacétique sont lentement additionnés. Après 2 heures d'agitation à température ambiante, le milieu est dilué avec 100 mL de toluène et concentré sous pression réduite. Le résidu obtenu est cristallisé dans l'éther diéthylique pour donner une poudre qui est filtrée, rincée à l'éther diéthylique et séchée sous vide. On obtient 1,675 g du produit attendu sous la forme d'une poudre rose hygroscopique.
LC-MS : M+H = 242
RMN-¹H (DMSO) δ (ppm): 8,75 (s large, 2H); 7,45 (t, 1H); 6,95 (d, 1H); 6,75 (d, 1H); 4,65 (qt, 1H); 4,00 (d large, 4H); 2,80 (m, 2H); 2,25 (m, 2H).

### 10.6. 6-(4-Chloro-3-fluoro-phénoxy)-2-aza-spiro[3.3]heptane-2-carboxylate de 3-carbamoyl-isoxazol-5-ylméthyle

On procède suivant le mode opératoire décrit dans l'exemple 7, étape 7.5.. A partir de 0,600 g (1,69 mmoles) de trifluoroacétate de 6-(4-chloro-3-fluoro-phénoxy)-2-azaspiro[3.3]heptane, obtenu à l'étape 10.5., de 0,622 g (2,02 mmoles) de 4-nitro-phényl-carbonate de 3-carbamoyl-isoxazol-5-ylméthyle, obtenu à l'étape 7.4., de 1,03 mL (5,90 mmoles) de N,N-diisopropyléthylamine et de 0,021 g (0,17 mmole) de N,N-diméthylaminopyridine, et après chromatographie sur gel de silice en éluant avec un mélange 99/1/0,1 puis 98/2/0,2 de dichlorométhane, de méthanol et d'ammoniaque à 30%, on obtient 0,602 g de produit attendu sous la forme d'une poudre blanche.
Point de fusion (°C) : 135-137°C
LC-MS : M+H = 410
RMN-¹H (DMSO) δ (ppm) : 8,15 (s, 1H); 7,85 (s, 1H); 7,45 (t, 1H); 6,95 (d, 1H); 6,80 (s, 1H); 6,75 (d, 1H); 5,20 (s, 2H); 4,65 (qt, 1H); 4,10-3,90 (d large, 4H); 2,75 (m, 2H); 2,25 (m, 2H).

### Exemple 11 (Composé N°25)

### 6-(4'-Fluoro-biphényl-4-yloxy)-2-aza-spiro[3.3]heptane-2-carboxylate de 3-méthylcarbamoyl-isoxazol-5-ylméthyle

### 11.1. 6-(4'-Fluoro-biphényl-4-yloxy)-2-azaspiro[3.3]heptane-2-carboxylate de tert-butyle

On procède suivant le mode opératoire décrit dans l'exemple 2, étape 2.1.. A partir de 0,900 g (4,22 mmoles) de 6-hydroxy-2-aza-spiro[3.3]heptane-2-carboxylate de tert-butyle, obtenu à l'étape 10.3., de 0,953 g (5,06 mmoles) de 4'-fluoro-biphényl-4-ol, de 0,845 g (4,85 mmoles) de diéthylazodicarboxylate et de 1,328 g (5,06 mmoles) de triphénylphosphine, et après chromatographie sur gel de silice en éluant avec un mélange de 95/5 puis 90/10 de cyclohexane et d'acétate d'éthyle, on obtient 1,44 g de produit attendu sous la forme d'une poudre beige utilisée telle quelle dans l'étape suivante.

### 11.2. Trifluoroacétate de 6-(4'-fluoro-biphényl-4-yloxy)-2-aza-spiro[3.3]heptane

On procède suivant le mode opératoire décrit dans l'exemple 10, étape 10.5. A partir de 1,44 g (3,76 mmoles) de 6-(4'-fluoro-biphényl-4-yloxy)-2-aza-spiro[3.3]heptane-2-carboxylate de tert-butyle, obtenu à l'étape 11.1., et de 8 mL (105,24 mmoles) d'acide trifluoroacétique, on obtient 1,04 g de produit attendu sous la forme d'une poudre hygroscopique.
LC-MS : M+H = 284
RMN-¹H (DMSO) δ (ppm) : 8, 60 (s large, 1H) ; 7, 65 (t, 2H) ; 7,60 (d, 2H); 7,25 (t, 2H); 6,90 (d, 2H); 4,70 (qt, 1H); 4,05 (d large, 4H); 2,85 (m, 2H); 2,30 (m, 2H).

### 11.3. 6-(4'-Fluoro-biphényl-4-yloxy)-2-azaspiro[3.3]heptane-2-carboxylate de 3-méthylcarbamoyl-isoxazol-5-ylméthyle

On procède suivant le mode opératoire décrit dans l'exemple 7, étape 7.5.. A partir de 0,500 g (1,26 mmoles) de trifluoroacétate de 6-(4'-fluoro-biphényl-4-yloxy)-2-azaspiro[3.3]heptane, obtenu à l'étape 11.2., de 0,485 g (1,51 mmoles) de 4-nitro-phényl-carbonate de 3-méthylcarbamoylisoxazol-5-ylméthyle, obtenu à l'étape 6.1, de 0,77 mL (4,40 mmoles) de N,N-diisopropyléthylamine et de 0,015 g (0,13 mmole) de N,N- diméthylaminopyridine, et après chromatographie sur gel de silice en éluant avec un mélange 99/1/0,1 de dichlorométhane, de méthanol et d'ammoniaque à 30%, on obtient 0,538 g de produit attendu sous la forme d'une poudre blanche.
Point de fusion (°C) : 163-165°C
LC-MS : M+H = 466
RMN-¹H (DMSO) δ (ppm) : 8,70 (qd, 1H) ; 7,65 (t, 2H) ; 7,55 (d, 2H); 7,25 (t, 2H); 6,90 (d, 2H); 6,80 (s, 1H); 5,20 (s, 2H); 4,65 (qt, 1H); 4,10-3,90 (d large, 4H); 2,80 (d, 3H); 2,75 (m, 2H); 2,25 (m, 2H).

Le tableau 1 qui suit illustre les structures chimiques et les propriétés physiques de quelques composés selon l'invention.
Dans ce tableau, les composés sont sous forme de base libre ou de sel.

**Tableau 1**

| | | | | | | | | | | |
|---|---|---|---|---|---|---|---|---|---|---|
| 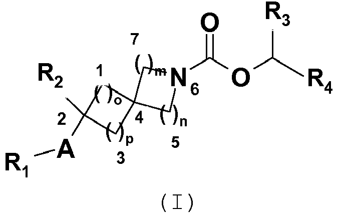 | | | | | | | | | | |

| **N°** | **R₁** | **m** | **n** | **o** | **p** | **A** | **R₂** | **R₃** | **R₄** | **Base ou sel** |
|---|---|---|---|---|---|---|---|---|---|---|
| **1.** | 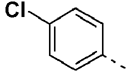 | **2** | **2** | **1** | **1** | **O** | **H** | **H** | 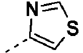 | **base** |
| **2.** | 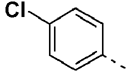 | **2** | **2** | **1** | **1** | **O** | **H** | **H** | 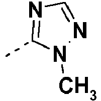 | **HCl** |
| **3.** | 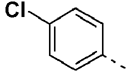 | **2** | **2** | **1** | **1** | **O** | **H** | **H** | 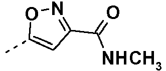 | **base** |
| **4.** | 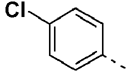 | **2** | **2** | **1** | **1** | **O** | **H** | **H** | 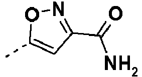 | **base** |
| **5.** | 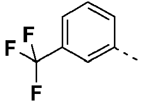 | **2** | **2** | **1** | **1** | **O** | **H** | **H** | 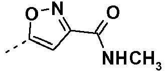 | **base** |
| **6.** | 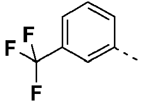 | **2** | **2** | **1** | **1** | **O** | **H** | **H** | 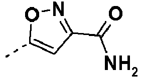 | **base** |
| **7.** | 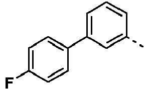 | **2** | **2** | **1** | **1** | **O** | **H** | **H** | 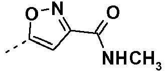 | **base** |
| **8.** | 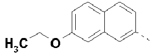 | **2** | **2** | **1** | **1** | **O** | **H** | **H** | 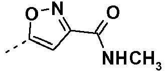 | **base** |
| **9.** | 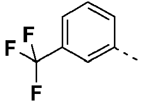 | **2** | **1** | **1** | **1** | **O** | **H** | **H** | 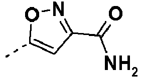 | **HCl** |
| **10.** | 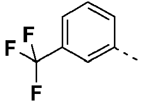 | **2** | **1** | **1** | **1** | **O** | **H** | **H** | 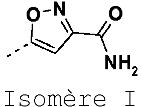 | **base** |
| **11.** | 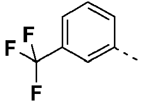 | **2** | **1** | **1** | **1** | **O** | **H** | **H** | 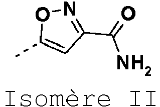 | **base** |
| **12.** | 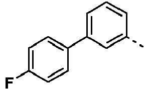 | **2** | **2** | **1** | **1** | **O** | **H** | **H** | 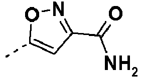 | **base** |
| **13.** | 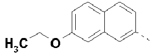 | **2** | **2** | **1** | **1** | **O** | **H** | **H** | 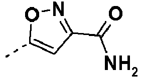 | **base** |
| **14.** | 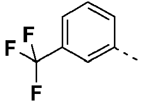 | **2** | **1** | **1** | **1** | **O** | **H** | **H** | 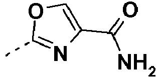 | **base** |
| **15.** | 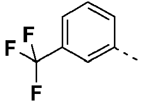 | **2** | **1** | **1** | **1** | **O** | **H** | **H** | 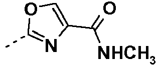 | **base** |
| **16.** | 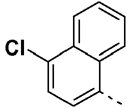 | **2** | **2** | **1** | **1** | **O** | **H** | **H** | 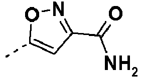 | **base** |
| **17.** | 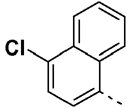 | **2** | **2** | **1** | **1** | **O** | **H** | **H** | 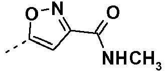 | **base** |
| **18.** | 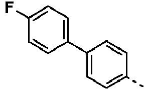 | **2** | **2** | **1** | **1** | **O** | **H** | **H** | 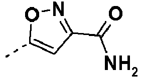 | **base** |
| **19.** | 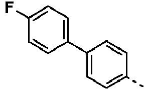 | **2** | **2** | **1** | **1** | **O** | **H** | **H** | 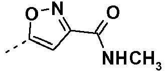 | **base** |
| **20.** | 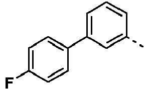 | **2** | **2** | **1** | **1** | **O** | **H** | **H** | 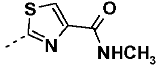 | **base** |
| **21.** | 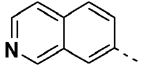 | **2** | **2** | **1** | **1** | **O** | **H** | **H** | 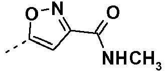 | **base** |
| **22.** | 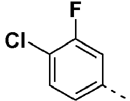 | **2** | **2** | **1** | **1** | **O** | **H** | **H** | 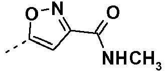 | **base** |
| **23.** | 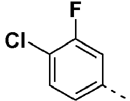 | **1** | **1** | **1** | **1** | **O** | **H** | **H** | 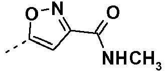 | **base** |
| **24.** | 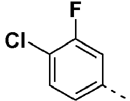 | **1** | **1** | **1** | **1** | **O** | **H** | **H** | 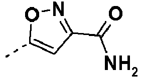 | **base** |
| **25.** | 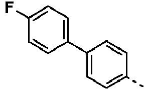 | **1** | **1** | **1** | **1** | **O** | **H** | **H** | 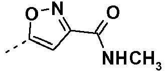 | **base** |
| **26.** | 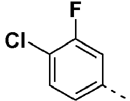 | **2** | **1** | **1** | **1** | **O** | **H** | **H** | 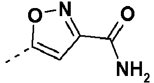 | **base** |
| **27.** | 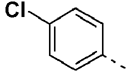 | **2** | **1** | **1** | **1** | **O** | **H** | **H** | 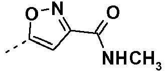 | **base** |
| **28.** | 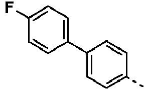 | **2** | **1** | **1** | **1** | **O** | **H** | **H** | 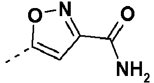 | **base** |
| **29.** | 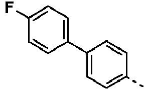 | **2** | **1** | **1** | **1** | **O** | **H** | **H** | 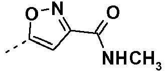 | **base** |
| **30.** | 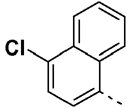 | **2** | **1** | **1** | **1** | **O** | **H** | **H** | 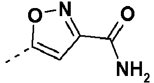 | **base** |
| **31.** | 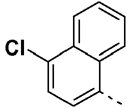 | **2** | **1** | **1** | **1** | **O** | **H** | **H** | 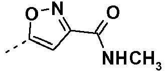 | **base** |
| **32.** | 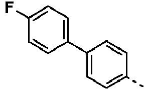 | **2** | **1** | **1** | **1** | **O** | **H** | **H** | 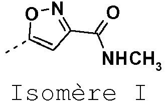 | **base** |
| **33.** | 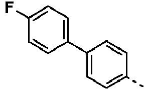 | **2** | **1** | **1** | **1** | **O** | **H** | **H** | 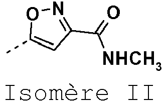 | **base** |

Le tableau 2 qui suit donne les résultats des analyses RMN ¹H, les points de fusions (PF), et les masses M+H mesurées pour les composés du tableau 1.

**Tableau 2**

| ***N°*** | ***RMN 1H 400Mhz DMSO*/*CDCl₃*** | **MP** | **M+H** |
|---|---|---|---|
| **1** | δ (ppm) CDCl₃: 8,85 (s, 1H); 7,40 (s, 1H); 7,30 (t, 2H) ; 6,80 (d, 2H) ; 5,35 (s, 2H) ; 4,70 (qt, 1H); 3,35 (m, 4H); 2,45 (m, 2H); 2,00 (m, 2H) ; 1,65 (m, 4H). | 86-88°C | 393 |
| **2** | 7,8 (d, 1H) ; 7,75 (d, 1H) ; 7,35 (d, 2H) 6,90 (d, 2H) ; 5,35 (s, 2H) ; 4,95 (m, 2H) ; 3,55 (m, 2H) ; 3,25 (large m, 2H) ; 2,85 (large m , 2H) ; 2,0 (m, 2H) ; 1,85 (m, 2H). | 163-165°C | 391 |
| **3** | δ (ppm) DMSO: 8,85 (s, 1H); 7,35 (d, 2H); 6,90 (d, 2H); 6,80 (s, 1H); 5,25 (s, 2H); 4,75 (qt, 1H); 3,35 (d large, 4H); 2,80 (s, 3H); 2,45 (m, 2H); 1,80 (m, 2H); 1,55 (dt, 4H). | 147-149°C | 434 |
| **4** | δ (ppm) DMSO: 8,15 (s, 1H); 7,85 (s, 1H); 7,30 (d, 2H); 6,90 (d, 2H); 6,80 (s, 1H); 5, 25 (s, 2H); 4, 75 (qt, 1H); 3, 35 (d large, 4H); 2,45 (t, 2H); 1,80 (t, 2H); 1,55 (d, 4H). | 70-72°C | 420 |
| **5** | δ (ppm) DMSO: 8,70 (s, 1H); 7,45 (t, 1H); 7,30 (d, 1H); 7,15 (d, 1H); 7,10 (s, 1H); 6,80 (s, 1H); 5,25 (s, 2H); 4,85 (m, 1H); 3,35 (d large, 4H); 2,80 (s, 3H); 2,45 (m, 2H); 1,85 (m, 2H); 1,55 (d, 4H). | 93-95°C | 468 |
| **6** | δ (ppm) DMSO: 8,10 (s, 1H); 7,85 (s, 1H); 7,50 (t, 1H); 7,25 (d, 1H); 7,15 (d, 1H); 7,10 (s, 1H); 6,80 (s, 1H); 5,25 (s, 2H); 4,85 (qt, 1H); 3,35 (d large, 4H); 2,45 (m, 2H); 1,85 (m, 2H); 1,55 (d, 4H). | 88-90°C | 454 |
| **7** | δ (ppm) DMSO: 8,70 (s, 1H); 7,70 (t, 2H); 7,40 (t, 1H); 7,30 (t, 2H); 7,20 (d, 1H); 7,10 (s, 1H); 6,85 (d, 1H); 6,80 (s, 1H); 5,25 (s, 2H); 4,85 (qt, 1H); 3,35 (d large, 4H); 2,80 (s, 3H); 2,50 (m, 2H); 1,85 (m, 2H); 1,55 (d, 4H). | 94-96°C | 494 |
| **8** | δ (ppm) DMSO: 8,70 (s large, 1H); 7,70 (d, 2H); 7,20 (s, 1H); 7,10 (s, 1H); 6,95 (t, 2H); 6,80 (s, 1H); 5,25 (s, 2H); 4,85 (qt, 1H); 4,15 (qd, 2H); 3,40 (d large, 4H); 2,80 (s, 3H); 2,55 (m, 2H); 1,90 (m, 2H); 1,60 (dt, 4H); 1,40 (t, 3H). | 73-75°C | 494 |
| **9** | δ (ppm) DMSO: 8,15 (s, 1H); 7,80 (s, 1H); 7,50 (t, 1H); 7,30 (d, 1H); 7,15 (m, 2H); 6,80 (d, 1H); 5,25 (d, 2H); 4,90 (m, 1H); 3,45 (s, 2H); 3,35 (m, 2H); 2,55 (m, 2H); 2,10 (m, 2H) ; 1,95 (m, 2H). | huile | 440 |
| **10** | δ (ppm) DMSO: 8,15 (s, 1H); 7,85 (s, 1H); 7,55 (t, 1H); 7,30 (d, 1H); 7,20 (d, 1H), 7,15 (s, 1H); 6,85 (d, 1H) ; 5,25 (s, 2H); 4,90 (qt, 1H); 3,45 (s, 2H); 3,30 (m, 2H); 2,55 (m, 2H); 2,05 (m, 2H); 1,95 (m, 2H). | 97-99°C | 440 |
| **11** | δ (ppm) DMSO: 8,10 (s, 1H); 7,85 (s, 1H); 7,55 (t, 1H); 7,30 (d, 1H); 7,20 (d, 1H), 7,15 (s, 1H); 6,80 (s, 1H); 5,25 (s, 2H); 4,85 (qt, 1H); 3,45-3,25 (m, 4H); 2,50 (m, 2H); 2,10 (m, 2H); 1,95 (m, 2H). | cire | 440 |
| **12** | δ (ppm) DMSO: 8,10 (s, 1H); 7,85 (s, 1H); 7,55 (t, 1H); 7,30 (d, 1H); 7,20 (d, 1H), 7,15 (s, 1H); 6,80 (s, 1H); 5,25 (s, 2H); 4,85 (qt, 1H); 3,45-3,25 (m, 4H); 2,50 (m, 2H); 2,10 (m, 2H); 1,95 (m, 2H). | 74-76°C | 480 |
| **13** | δ (ppm) DMSO: 8,10 (s, 1H); 7,85 (s, 1H); 7,55 (t, 1H); 7,30 (d, 1H); 7,20 (d, 1H), 7,15 (s, 1H); 6,80 (s, 1H); 5,25 (s, 2H); 4,85 (qt, 1H); 3,45-3,25 (m, 4H); 2,50 (m, 2H); 2,10 (m, 2H); 1,95 (m, 2H). | 114-116°C | 480 |
| **14** | (ppm) DMSO: 8,60 (d, 1H); 7,65 (s large, 1H); 7,50 (m, 2H); 7,30 (d, 1H); 7,15 (m, 2H); 5,20 (d, 2H); 4,90 (m, 1H); 3,45 (s, 2H); 3,35 (m, 2H); 2,55 (m, 2H); 2,10 (m, 2H); 1,95 (m, 2H). | 90-92°C | 440 |
| **15** | δ (ppm) DMSO: 8,60 (d, 1H); 8,25 (s large, 1H); 7,55 (t, 1H); 7,30 (d, 2H); 7,15 (m, 2H); 5,20 (d, 2H); 4,90 (m, 1H); 3,45 (s, 2H); 3,35 (m, 2H); 2,75 (s, 3H); 2,55 (m, 2H); 2,10 (m, 2H); 1,95 (m, 2H). | cire | 454 |
| **16** | δ (ppm) DMSO: 8,25 (d, 1H); 8,15 (d, 2H); 7,85 (s, 1H); 7,75 (t, 1H); 7,65 (t, 1H), 7,60 (d, 1H); 6,80 (d, 2H); 5,25 (s, 2H); 4,95 (qt, 1H); 3,40 (d large, 4H); 2,55 (m, 2H); 1,95 (m, 2H); 1,60 (dt, 4H). | 75-77°C | 468 |
| **17** | δ (ppm) DMSO: 8,70 (s, 1H); 8,25 (d, 1H); 8,15 (d, 1H); 7,75 (t, 1H); 7,65 (t, 1H), 7,60 (d, 1H); 6,80 (d, 2H); 5,25 (s, 2H); 4,95 (qt, 1H); 3,40 (d large, 4H); 2,80 (s, 3H); 2,55 (m, 2H); 1,95 (m, 2H); 1,60 (dt, 4H). | 62-64°C | 484 |
| **18** | δ (ppm) DMSO: 8,15 (s, 1H); 7,85 (s, 1H); 7,65 (t, 2H); 7,55 (d, 2H); 7,25 (t, 2H), 6,95 (d, 2H); 6,80 (s, 1H) ; 5,25 (s, 2H) ; 4,80 (qt, 1H); 3,35 (d large, 4H); 2,45 (m, 2H); 1,85 (m, 2H); 1,60 (dt, 4H). | 163-165°C | 497* |
| **19** | δ (ppm) DMSO: 8,70 (s, 1H); 7,65 (t, 2H); 7,55 (d, 2H); 7,25 (t, 2H), 6,95 (d, 2H); 6,80 (s, 1H); 5,25 (s, 2H); 4,80 (qt, 1H); 3,35 (d large, 4H); 2,80 (s, 3H); 2,45 (m, 2H); 1,85 (m, 2H); 1,60 (dt, 4H). | 174-176°C | 494 |
| **20** | δ (ppm) DMSO: 8,35 (s large, 1H); 8,25 (s, 1H); 7,70 (t, 2H); 7,35 (t, 1H); 7,30 (t, 2H); 7,20 (d, 1H); 7,05 (s, 1H); 6,85 (d, 1H); 5,35 (s, 2H); 4,90 (qt, 1H); 3,40 (d large, 4H); 2,80 (s, 3H); 2,50 (m, 2H); 1,85 (m, 2H); 1,60 (dt, 4H). | 130-132°C | 510 |
| **21** | δ (ppm) DMSO: 9,20 (s, 1H); 8,70 (s large, 1H); 8,40 (d, 1H); 7,90 (d, 1H); 7,75 (d, 1H); 7,40 (t, 2H); 6,80 (s, 1H); 5,25 (s, 2H); 4,95 (qt, 1H); 3,40 (d large, 4H); 2,80 (s, 3H); 2,60 (m, 2H); 1,90 (m, 2H); 1,60 (dt, 4H). | 79-81°C | 451 |
| **22** | δ (ppm) DMSO: 8,70 (s large, 1H); 7,45 (t, 1H); 6,95 (d, 1H); 6,80 (s, 1H); 6,75 (d, 1H); 5,25 (s, 2H); 4,80 (qt, 1H); 3,35 (d large, 4H); 2,80 (s, 3H); 2,45 (m, 2H); 1,85 (m, 2H); 1,55 (dt, 4H). | 107-108°C | 452 |
| **23** | δ (ppm) DMSO: 8,70 (qd, 1H); 7,45 (t, 1H); 6,90 (d, 1H); 6,80 (s, 1H); 6,75 (d, 1H); 5,20 (s, 2H); 4,65 (qt, 1H); 4,10-3,90 (d large, 4H); 2,80 (d, 3H); 2,75 (m, 2H); 2,25 (m, 2H). | 84-86°C | 424 |
| **24** | δ (ppm) DMSO: 8,15 (s, 1H); 7,85 (s, 1H); 7,45 (t, 1H); 6,95 (d, 1H); 6,80 (s, 1H); 6,75 (d, 1H); 5,20 (s, 2H); 4,65 (qt, 1H); 4,10-3,90 (d large, 4H); 2,75 (m, 2H); 2,25 (m, 2H). | 135-137°C | 410 |
| **25** | δ (ppm) DMSO: 8,70 (qd, 1H); 7,65 (t, 2H); 7,55 (d, 2H); 7,25 (t, 2H); 6,90 (d, 2H); 6,80 (s, 1H); 5,20 (s, 2H); 4,65 (qt, 1H); 4,10-3,90 (d large, 4H); 2,80 (d, 3H); 2,75 (m, 2H); 2,25 (m, 2H). | 163-165°C | 466 |
| **26** | δ (ppm) DMSO: 8,15 (s, 1H); 7,85 (s, 1H); 7,45 (t, 1H); 7,00 (d, 1H); 6,80 (m, 2H); 5,25 (d, 2H) ; 4,80 (m, 1H) ; 3,35 (m, 4H) ; 2,55 (m, 2H) ; 2,10 (m, 2H) ; 1,95 (m, 2H). | 83-92°C | 424 |
| **27** | δ (ppm) DMSO: 8,70 (s large, 1H); 7,30 (d, 2H); 6,90 (d, 2H); 6,80 (d, 1H); 5,25 (d, 2H); 4,75 (m, 1H); 3,35 (m, 4H); 2,80 (s, 3H); 2,50 (m, 2H); 2,05 (m, 2H); 1,95 (m, 2H). | 94-97°C | 420 |
| **28** | δ (ppm) DMSO: 8,15 (s, 1H); 7,85 (s, 1H); 7,65 (t, 2H); 7,55 (d, 2H); 7,25 (t, 2H); 6,95 (d, 2H); 6,80 (d, 1H); 5,25 (d, 2H); 4,85 (m, 1H); 3,45 (d, 2H); 3,35 (m, 2H); 2,55 (m, 2H); 2,10 (m, 2H); 1,95 (m, 2H). | 131-133°C | 466 |
| **29** | δ (ppm) DMSO: 8,70 (s large, 1H); 7,65 (t, 2H); 7,55 (d, 2H); 7,25 (t, 2H); 6,95 (d, 2H); 6,80 (d, 1H); 5,25 (d, 2H); 4,80 (m, 1H); 3,35 (m, 4H); 2,80 (s, 3H); 2,50 (m, 2H); 2,10 (m, 2H); 1,95 (m, 2H). | 135-137°C | 480 |
| **30** | δ (ppm) DMSO: 8,25 (d, 1H); 8,15 (d, 2H); 7,85 (s large, 1H); 7,75 (t, 1H); 7,65 (t, 1H); 7,55 (d, 1H); 6,85 (d, 1H); 6,80 (s, 1H); 5,25 (d, 2H); 5,00 (m, 1H); 3,40 (m, 4H); 2,65 (m, 2H); 2,20 (m, 2H); 2,00 (m, 2H). | 149-150°C | 456 |
| **31** | δ (ppm) DMSO: 8,70 (s, 1H); 8,25 (d, 1H); 8,15 (d, 1H); 7,75 (t, 1H); 7,65 (t, 1H); 7,55 (d, 1H); 6,85 (d, 1H); 6,80 (s, 1H); 5,25 (d, 2H); 5,00 (m, 1H); 3,40 (m, 4H); 2,80 (s, 3H); 2,60 (m, 2H); 2,20 (m, 2H); 2,00 (m, 2H). | 87-92°C | 470 |
| **32** | δ (ppm) DMSO: 8,70 (s, 1H); 7,65 (t, 2H); 7,55 (d, 2H); 7,25 (t, 2H), 6,95 (d, 2H); 6,80 (s, 1H); 5,25 (s, 2H); 4,80 (qt, 1H); 3,35 (m, 4H); 2,80 (s, 3H); 2,50 (t, 2H); 2, 15 (t, 2H) ; 1,95 (m, 2H). | 149-151°C | 480 |
| **33** | δ (ppm) DMSO: 8,70 (s large, 1H); 7,65 (t, 2H); 7,55 (d, 2H); 7,25 (t, 2H), 6,95 (d, 2H); 6,80 (d, 1H); 5,25 (s, 2H); 4,85 (qt, 1H); 3,45 (m, 2H); 3,35 (m, 2H) ; 2,80 (s, 3H); 2,50 (m, 2H); 2,10 (t, 2H); 1,95 (m, 2H). | 150-152°C | 480 |

| | | | |
|---|---|---|---|
| * Pic M+NH4+ observé. | | | |

Le tableau 3 ci-après fait figurer les proportions relatives et temps de rétention des isomères des composés 9, 14, 15, 26, 27, 28, 29, 30 et 31, obtenus à partir d'une des deux méthodes d'analyse chromatographique ci-après :

### Méthode 1 :

HPLC / ZQ - Gradient 10 min
Phases mobiles: Phase A: CH3COONH4 + 3% ACN Phase B: ACN
Phase stationnaire / colonne: Colonne Kromasil C18,
Dimensions: 50*2,1mm ; 3,5µm
Débit: D= 0,8 mL/min
Température de la colonne: T= 40 °C
Volume d'injection: V= 5µL
Gradient: T= 0min : 100% de A, de T= 5,5min à T= 7min: 100% de B, de T= 7,1min à T= 10min: 100% de A

### Méthode 2 :

UPLC / TOF - Gradient 3 min
Phases mobiles: Phase A: H2O + 0,05% de TFA
   Phase B: ACN + 0,035% de TFA
Phase stationnaire / colonne: Colonne Acquity BEH C18,
Dimensions: 50*2,1mm ; 1,7µm
Débit:D= 1,0 mL/min
Température de la colonne: T= 40 °C
Volume d'injection: V= 2µL
Gradient:T= 0min : 98% de A et 2% de B, de T= 1,6min à T= 2,1min: 100% de B, de T= 2,5min à T= 3min: 98% de A et 2% de B.

**Tableau 3**

| N° | méthode | MH+ | Isomère I (temps de rétention/pureté UV) | Isomère II (temps de rétention/pureté UV) |
|---|---|---|---|---|
| 9 | 1 | 440 | tr=1.20min/42,22 % | tr=1,22min/51.52% |
| 14 | 2 | 440 | tr=1.15min/46.70 % | tr=1,17min/52.00% |
| 15 | 2 | 454 | tr=1.19min/45.66 % | tr=1.22min/51.46% |
| 26 | 2 | 424 | tr=1.16min/60.02 % | tr=1.19min/36.63% |
| 27 | 2 | 420 | tr=1.22min/52.47 % | tr=1.24min/47.53% |
| 28 | 2 | 466 | tr=1.26min/51.60 % | tr=1.28min/47.34% |
| 29 | 2 | 480 | tr=1.30min/51.76 % | tr=1.32min/47.13% |
| 30 | 1 | 456 | tr=4.80min/50.99 % | tr=4.90min/46.53% |
| 31 | 1 | 470 | tr=4.92min/52.70 % | tr=5.02min/45.90% |

Les composés de l'invention ont fait l'objet d'essais pharmacologiques permettant de déterminer leur effet inhibiteur de l'enzyme FAAH (Fatty Acid Amide Hydrolase).

### Protocole 1

L'activité inhibitrice a été mise en évidence dans un test radioenzymatique basé sur la mesure du produit d'hydrolyse de l'anandamide [éthanolamine 1-³H] par la FAAH (Life Sciences (1995), 56, 1999-2005 et Journal of Biochemical and Biophysical Methods (2004), 60(2), 171-177). Ainsi, les cerveaux de souris (moins le cervelet) sont prélevés et conservés à -80°C. Les homogénats membranaires sont préparés extemporanément par homogénéisation des tissus à l'aide d'un appareil Precellys® dans le tampon de réaction (Tris-HCl 10 mM pH=8, NaCl 150 mM et acide éthylène-diamine-tétraacétique (EDTA) 1 mM). La réaction enzymatique est conduite dans des plaques de filtration Multiscreen 96puits dans un volume final de 70µl. Du tampon de réaction supplémenté avec de l'albumine de sérum bovin sans acides gras (BSA, 1 mg/ml) est utilisé pour la réaction enzymatique, la dilution des composés et de l'anandamide [éthanolamine 1-³H]. Sont ajoutés successivement dans les puits, du tampon de réaction contenant la BSA (43µl/puits), les composés dilués testés à différentes concentrations (7µl/puits contenant 1% de DMSO) et la préparation membranaire (10µl/puits soit 200 µg de tissu par essai). Après 20 minutes de pré-incubation des composés avec l'enzyme à 25°C, la réaction est démarrée par l'addition d'anandamide [éthanolamine 1-³H] (activité spécifique de 15-20 Ci/mmol) diluée avec de l'anandamide froide (10µl/puits, concentration finale de 10 µM, 0,01µCi par essai). Après 20 minutes d'incubation à 25°C, la réaction enzymatique est arrêtée par addition d'une solution de charbon actif 5M préparée dans un tampon NaCl 1,5M et HCl 0,5M (50µl/puits). Le mélange est agité 10 minutes puis la phase aqueuse contenant l'éthanolamine [1-³H] est récupérée par filtration sous-vide et comptée par scintillation liquide.

### Protocole 2

L'activité inhibitrice a été mise en évidence par technique fluorescente dans un test enzymatique basé sur la mesure du produit d'hydrolyse fluorescent de l'arachidonoyl 7-amino 4-méthyl coumarin amide (AAMC) par la FAAH (Analytical Biochemistry (2005), 343:143-151, J. of Biomolecular Screening (2006), 11(5): 519-527 et J. of Neurosciences Methods (2007), 161: 47-54). Ainsi, les cerveaux de souris (moins le cervelet) sont prélevés et conservés à -80°C. Les homogénats de cerveaux sont préparés extemporanément par homogénéisation des tissus à l'aide d'un appareil Precellys® dans le tampon de réaction (Tris-HCl 10 mM pH=8, NaCl 150 mM et acide éthylène-diamine-tétraacétique (EDTA) 1 mM). La réaction enzymatique est conduite dans des plaques 384 puits noires en polystyrène dans un volume final de 50µL. Du tampon de réaction supplémenté avec de l'albumine de sérum bovin sans acides gras (BSA, 1 mg/ml) est utilisé pour la réaction enzymatique, la dilution des composés et la dilution de l'AAMC. Sont ajoutés successivement dans les puits, du tampon de réaction contenant la BSA (25µl/puits), les composés dilués testés à différentes concentrations (5µl/puits contenant 1% de DMSO) et la préparation membranaire (10µl/puits soit 200 µg de tissu par essai). Après 20 minutes de pré-incubation des composés avec l'enzyme à 25°C, la réaction est démarrée par l'addition de 10µL de substrat par puits (AAMC, concentration finale de 10 µM). Après 40 minutes d'incubation à 37°C, l'aminométhyl coumarin (AMC) produit est mesuré par comptage fluorescent (lecteur de plaque Envision).

Dans les conditions du protocole 1, les composés les plus actifs de l'invention présentent des CI₅₀ (concentration inhibant de 50 % l'activité enzymatique contrôle de la FAAH) comprises entre 0,001 et 1 µM. Par exemple les composés n°7, 29, 32 et 33 ont des CI₅₀ respectives de 19 nM, 5,3 nM, 3 nM et 19 nM.

Dans les conditions du protocole 2, les composés les plus actifs de l'invention présentent des CI₅₀ (concentration inhibant de 50 % l'activité enzymatique contrôle de la FAAH) comprises entre 0,001 et 1 µM. Par exemple les composés n°19 et n°25 ont des CI₅₀ respectives de 1,7 nM et 0,46 nM.

Il apparaît donc que les composés selon l'invention ont une activité inhibitrice sur l'enzyme FAAH.

L'activité *in vivo* des composés de l'invention peut être évaluée dans un test d'analgésie.
Ainsi, l'administration intrapéritonéale (i.p.) de PBQ (phénylbenzoquinone, 2 mg/kg dans une solution de chlorure de sodium à 0,9 % contenant 5 % d'éthanol) chez des souris mâles OF1 de 25 à 30 g, provoque des étirements abdominaux, en moyenne 30 torsions ou contractions pendant la période de 5 à 15 minutes après injection. Les composés testés sont administrés par voie orale (p.o.) ou par voie intrapéritonéale (i.p.) en suspension dans du Tween 80 à 0,5 %, 60 minutes ou 120 minutes avant l'administration de PBQ. Dans ces conditions, les composés les plus puissants réduisent de 35 à 80 % le nombre d'étirements induits par le PBQ, dans une gamme de doses comprise entre 1 et 30 mg/kg. Par exemple, le composé n°25 du tableau 1 réduit de 50% le nombre d'étirements induits par le PBQ, à la dose de 30 mg/kg p.o. à 120 minutes.

L'enzyme FAAH *(*Chemistry and Physics of Lipids, (2000), 108, 107-121) catalyse l'hydrolyse des dérivés endogènes d'amides et d'esters de différents acides gras tels que la N-arachidonoyléthanolamine (anandamide), la N-palmitoyl-éthanolamine, la N-oléoyléthanolamine, l'oléamide ou le 2-arachidonoylglycérol. Ces dérivés exercent différentes activités pharmacologiques en interagissant, entre autres, avec les récepteurs cannabinoïdes et vanilloïdes.
Les composés de l'invention, bloquent cette voie de dégradation et augmentent le taux tissulaire de ces substances endogènes. Ils peuvent être utilisés à ce titre dans la prévention et le traitement des pathologies dans lesquelles les cannabinoïdes endogènes et/ou tous autres substrats métabolisés par l'enzyme FAAH, sont impliqués. On peut par exemple citer les maladies et les affections suivantes :
la douleur notamment les douleurs aiguës ou chroniques de type neurogène : migraine, douleurs neuropathiques incluant les formes associées au virus de l'herpès et au diabète et à la chimiothérapie, les douleurs aiguës ou chroniques associées aux maladies inflammatoires : arthrite, arthrite rhumatoïde, ostéoarthrite, spondylite, goutte, vascularite, maladie de Crohn, syndrome du colon irritable, les douleurs aiguës ou chroniques périphériques, les vertiges, les vomissements, les nausées en particulier celles consécutives à une chimiothérapie, les troubles du comportement alimentaire en particulier les anorexies et cachexies de diverses natures , les pathologies neurologiques et psychiatriques : tremblements, dyskinésies, dystonies, spasticité, comportements compulsifs et obsessionnels, syndrome de Tourette, toutes les formes de dépression et d'anxiété de toute nature et origine, troubles de l'humeur, psychoses, les maladies neuro-dégénératives aiguës et chroniques : maladie de Parkinson, maladie d'Alzheimer, démence sénile, chorée de Huntington, lésions liées à l'ischémie cérébrale et aux traumatismes crâniens et médullaires, l'épilepsie, les troubles du sommeil incluant les apnées du sommeil, les maladies cardiovasculaires en particulier hypertension, arythmies cardiaques, artériosclérose, crise cardiaque, ischémies cardiaques, l'ischémie rénale, les cancers : tumeurs bénignes de la peau, papillomes et tumeurs cérébrales, tumeurs de la prostate, tumeurs cérébrales (glioblastomes, médulloépithéliomes, médulloblastomes, neuroblastomes, tumeurs d'origine embryonnaire, astrocytomes, astroblastomes, épendyomes, oligodendrogliomes, tumeur du plexus, neuroépithéliomes, tumeur de l'épiphyse, épendymoblastomes, méningiomes malins, sarcomatoses, mélanomes malins, schwénnomes), les désordres du système immunitaire, notamment les maladies auto-immunes : psoriasis, lupus érythémateux, maladies du tissu conjonctif ou connectivités, syndrome de Sjögrer's, spondylarthrite ankylosante, spondylarthrite indifférenciée, maladie de Behcet's, anémies auto-immunes hémolytiques, sclérose en plaques, sclérose latérale amyotrophique, amyloses, rejet de greffes, maladies affectant la lignée plasmocytaire, les maladies allergiques : l'hypersensibilité immédiate ou retardée, rhinites ou conjonctivites allergiques, dermatites de contact, les maladies infectieuses parasitaires, virales ou bactériennes : SIDA, méningites, les maladies inflammatoires, notamment les maladies articulaires : arthrite, arthrite rhumatoïde, ostéoarthrite, spondylite, goutte, vascularite, maladie de Crohn, syndrome du colon irritable, l'ostéoporose, les affections oculaires : hypertension oculaire, glaucome, les affections pulmonaires : maladies des voies respiratoires, bronchospasmes, toux, asthme, bronchite chronique, obstruction chronique des voies respiratoires, emphysème, les maladies gastro-intestinales: syndrome du colon irritable, désordres inflammatoires intestinaux, ulcères, diarrhées, l'incontinence urinaire et l'inflammation vésicale.

L'utilisation des composés selon l'invention, à l'état de base, de sel d'addition à un acide, d'hydrate ou de solvat pharmaceutiquement acceptable, pour la préparation d'un médicament destiné à traiter les pathologies ci-dessus mentionnées fait partie intégrante de l'invention.

L'invention a également pour objet des médicaments qui comprennent un composé de formule (I), ou un sel d'addition à un acide, ou encore un hydrate ou un solvat pharmaceutiquement acceptable du composé de formule (I). Ces médicaments trouvent leur emploi en thérapeutique, notamment dans le traitement des pathologies ci-dessus mentionnées.

Selon un autre de ses aspects, la présente invention concerne des compositions pharmaceutiques renfermant en tant que principe actif, au moins un composé selon l'invention. Ces compositions pharmaceutiques contiennent une dose efficace d'un composé selon l'invention, ou un sel d'addition à un acide, ou un hydrate, ou un solvat pharmaceutiquement acceptable dudit composé, et éventuellement un ou plusieurs excipients pharmaceutiquement acceptables.
Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intrathécale, intranasale, transdermique, pulmonaire, oculaire ou rectale, le principe actif de formule (I) ci-dessus, ou son sel d'addition à un acide, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules, les chewing-gums et les solutions ou suspensions orales, les formes d'administration sublinguale, buccale, intratrachéale, intraoculaire, intranasale, par inhalation, les formes d'administration sous-cutanée, intramusculaire ou intraveineuse et les formes d'administration rectale ou vaginale. Pour l'application topique, on peut utiliser les composés selon l'invention dans des crèmes, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,01 à 20 mg de principe actif par kg de poids corporel, selon la forme galénique.
Il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

L'invention selon un autre de ses aspects, concerne également une méthode de traitement des pathologies ci-dessus indiquées qui comprend l'administration d'une dose efficace d'un composé selon l'invention, d'un de ses sels d'addition à un acide pharmaceutiquement acceptable, d'un solvat ou d'un hydrate dudit composé.

## Revendications

1. Composé répondant à la formule (I) 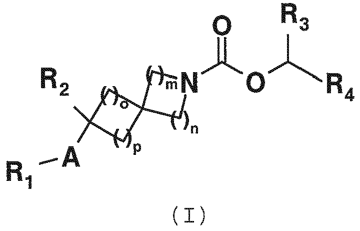 dans laquelle
R₂ représente un atome d'hydrogène, de fluor ou un groupe hydroxyle, cyano, trifluorométhyle, C₁₋₆- ,C₁₋₆-alcoxy, NR₈R₉ ;
m, n, o et p ont pour valeur 1, ou bien, p et o ont pour valeur 1, n et m ont pour valeur 2, ou bien n, o et p ont pour valeur 1 et m a pour valeur 2;A représente un atome d'oxygène ;
R₁ représente un groupe R₅ éventuellement substitué par un ou plusieurs groupes R₆ et/ou R₇ ;
R₅ représente un groupe choisi parmi un phényle, pyridinyle, pyridazinyle, pyrimidinyle, pyrazinyle, triazinyle, naphtalènyle, quinolinyle, isoquinolinyle, phtalazinyle, quinazolinyle, quinoxalinyle, cinnolinyle, naphthyridinyle ;
R₆ représente un atome d'halogène, un groupe cyano, -CH₂CN, nitro, hydroxyle, C₁₋₆-alkyle, C₁₋₆-alcoxy, C₁₋₆-thioalkyle, C₁₋₆-haloalkyle, C₁₋₆-haloalcoxy, C₁₋₆-halothioalkyle, C₃₋₇-cycloalkyle, C₃₋₇-cycloalkyle-C₁₋₃alkylène, C₃₋₇-cycloalkyle-C₁₋₃-alkylène-O-, NR₈R₉, NR₈COR₉, NR₈CO₂R₉, NR₈SO₂R₉, NR₈SO₂NR₈R₉, COR₈, CO₂R₈, CONR₈R₉, SO₂R₈, SO₂NR₈R₉ ou -O- (C₁₋₃alkylène) -O- ;
R₇ représente un groupe choisi parmi un phényle, pyridinyle, pyridazinyle, pyrimidinyle, pyrazinyle, triazinyle; le ou les groupes R₇ pouvant être substitués par un ou plusieurs groupes R₆ identiques ou différents l'un de l'autre ;
R₃ représente un atome d'hydrogène, de fluor, un groupe C₁₋₆-alkyle ou un groupe trifluorométhyle ;
R₄ représente un groupe choisi parmi un thiazolyle, un triazolyle, un oxazolyle et un isoxazolyle; ce groupe étant non substitué ou substitué par un ou plusieurs groupes C₁₋₆-alkyle ou CONR₈R₉ ;
R₈ et R₉ représentent indépendamment l'un de l'autre un atome d'hydrogène ou un groupe C₁₋₆-alkyle;
à l'état de base ou de sel d'addition à un acide.

2. Composé de formule (I) selon la revendication 1 **caractérisé en ce que** R₂ représente un atome d'hydrogène;
à l'état de base ou de sel d'addition à un acide.

3. Composé de formule (I) selon la revendication 1 ou 2 **caractérisé en ce que** R₁ représente un groupe R₅ non substitué ou substitué par un ou plusieurs groupes R₆ et/ou R₇ ;
R₅ représente un groupe phényle, naphtalènyle ou isoquinolinyle ;
R₆ représente un atome d'halogène, plus particulièrement un atome de fluor ou de chlore, ou un groupe C₁₋₆-haloalkyle, plus particulièrement trifluorométhyle, ou un groupe C₁₋₆-alcoxy, plus particulièrement un groupe éthoxy;
R₇ représente un phényle pouvant être substitué par un ou plusieurs groupes R₆ identiques ou différents l'un de l'autre; à l'état de base ou de sel d'addition à un acide.

4. Composé de formule (I) selon l'une quelconque des revendications 1 à 3 **caractérisé en ce que** R₃ représente un atome d'hydrogène; à l'état de base ou de sel d'addition à un acide.

5. Composé de formule (I) choisi parmi :
2-((4-Chloro-phénoxy)-7-aza-spiro[3.5]nonane-7-carboxylate de thiazol-4-ylméthyle ;
2-((4-chloro-phénoxy)-7-aza-spiro[3.5]nonane-7-carboxylate de 2-méthyl-2H-[1,2,4]triazol-3-ylméthyle et son chlorhydrate ;
2-((4-Chloro-phénoxy)-7-aza-spiro[3.5]nonane-7-carboxylate de 3-méthylcarbamoyl-isoxazol-5-ylméthyle ;
2-((4-Chloro-phénoxy)-7-aza-spiro[3.5]nonane-7-carboxylate de 3-carbamoyl-isoxazol-5-ylméthyle ;
2-((3-Trifluorométhyl-phénoxy)-7-aza-spiro[3.5]nonane-7-carboxylate de 3-méthylcarbamoyl-isoxazol-5-ylméthyle ;
2-((3-Trifluorométhyl-phénoxy)-7-aza-spiro[3.5]nonane-7-carboxylate de 3-carbamoyl-isoxazol-5-ylméthyle ;
2-((4'-Fluoro-biphényl-3-yloxy)-7-aza-spiro[3.5]nonane-7-carboxylate de 3-méthylcarbamoyl-isoxazol-5-ylméthyle ;
2-((7-Ethoxy-naphthalèn-2-yloxy)-7-aza-spiro[3.5]nonane-7-carboxylate de 3-méthylcarbamoyl-isoxazol-5-ylméthyle ;
2-((3-trifluorométhyl-phénoxy)-6-aza-spiro[3.4]octane-6-carboxylate de 3-carbamoyl-isoxazol-5-ylméthyle et son chlorhydrate (isomères I + II) ;
2-((3-Trifluorométhyl-phénoxy)-6-aza-spiro[3.4]octane-6-carboxylate de 3-carbamoyl-isoxazol-5-ylméthyle (isomère I du composé n°9) ;
2-((3-Trifluorométhyl-phénoxy)-6-aza-spiro[3.4]octane-6-carboxylate de 3-carbamoyl-isoxazol-5-ylméthyle (isomère II du composé n°9) ;
2-((4'-Fluoro-biphényl-3-yloxy)-7-aza-spiro[3.5]nonane-7-carboxylate de 3-carbamoyl-isoxazol-5-ylméthyle ;
2-((7-Ethoxy-naphthalèn-2-yloxy)-7-aza-spiro[3.5]nonane-7-carboxylate de 3-carbamoyl-isoxazol-5-ylméthyle ;
2-((3-Trifluorométhyl-phénoxy)-6-aza-spiro[3.4]octane-6-carboxylate de 4-carbamoyl-oxazol-2-ylméthyle (isomères I + II) ;
2-((3-Trifluorométhyl-phénoxy)-6-aza-spiro[3.4]octane-6-carboxylate de 4-méthylcarbamoyl-oxazol-2-ylméthyle (isomères I + II) ;
2-((4-Chloro-naphthalèn-1-yloxy)-7-aza-spiro[3.5]nonane-7-carboxylate de 3-carbamoyl-isoxazol-5-ylméthyle ;
2-((4-Chloro-naphthalèn-1-yloxy)-7-aza-spiro[3.5]nonane-7-carboxylate de 3-méthylcarbamoyl-isoxazol-5-ylméthyle ;
2-((4'-Fluoro-biphényl-4-yloxy)-7-aza-spiro[3.5]nonane-7-carboxylate de 3-carbamoyl-isoxazol-5-ylméthyle ;
2-((4'-Fluoro-biphényl-4-yloxy)-7-aza-spiro[3.5]nonane-7-carboxylate de 3-méthylcarbamoyl-isoxazol-5-ylméthyle ;
2-((4'-Fluoro-biphényl-3-yloxy)-7-aza-spiro[3.5]nonane-7-carboxylate de 4-méthylcarbamoyl-thiazol-2-ylméthyle ;
2-((Isoquinolin-7-yloxy)-7-aza-spiro[3.5]nonane-7-carboxylate de 3-méthylcarbamoyl-isoxazol-5-ylméthyle ;
2-((4-Chloro-3-fluoro-phénoxy)-7-aza-spiro[3.5]nonane-7-carboxylate de 3-méthylcarbamoyl-isoxazol-5-ylméthyle ;
6-((4-Chloro-3-fluoro-phénoxy)-2-aza-spiro[3.3]heptane-2-carboxylate de 3-méthylcarbamoyl-isoxazol-5-ylméthyle ;
6-((4-Chloro-3-fluoro-phénoxy)-2-aza-spiro[3.3]heptane-2-carboxylate de 3-carbamoyl-isoxazol-5-ylméthyle ;
6-((4'-Fluoro-biphényl-4-yloxy)-2-aza-spiro[3.3]heptane-2-carboxylate de 3-méthylcarbamoyl-isoxazol-5-ylméthyle ;
2-((4-Chloro-3-fluoro-phénoxy)-6-aza-spiro[3.4]octane-6-carboxylate de 3-carbamoyl-isoxazol-5-ylméthyle (isomères I + II) ;
2-((4-Chloro-phénoxy)-6-aza-spiro[3.4]octane-6-carboxylate de 3-méthylcarbamoyl-isoxazol-5-ylméthyle (isomères I + II) ;
2-((4'-Fluoro-biphényl-4-yloxy)-6-aza-spiro[3.4]octane-6-carboxylate de 3-carbamoyl-isoxazol-5-ylméthyle (isomères I + II) ;
2-((4'-Fluoro-biphényl-4-yloxy)-6-aza-spiro[3.4]octane-6-carboxylate de 3-méthylcarbamoyl-isoxazol-5-ylméthyle (isomères I + II);
2-((4-Chloro-naphthalèn-1-yloxy)-6-aza-spiro[3.4]octane-6-carboxylate de 3-carbamoyl-isoxazol-5-ylméthyle (isomères I + II) ;
2-((4-Chloro-naphthalèn-1-yloxy)-6-aza-spiro[3.4]octane-6-carboxylate de 3-méthylcarbamoyl-isoxazol-5-ylméthyle (isomères I + II);
2-((4'-Fluoro-biphényl-4-yloxy)-6-aza-spiro[3.4]octane-6-carboxylate de 3-méthylcarbamoyl-isoxazol-5-ylméthyle(isomère I du compose 29);
2-((4'-Fluoro-biphényl-4-yloxy)-6-aza-spiro[3.4]octane-6-carboxylate de 3-méthylcarbamoyl-isoxazol-5-ylméthyle(isomère II du composé 29).

6. Procédé de préparation d'un composé de formule (I) selon l'une quelconque des revendications 1 à 5, comprenant l'étape consistant à faire réagir soit une amine de formule générale (II), 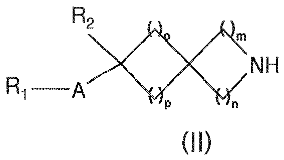 dans laquelle A, R₁, R₂, m, n, o et p sont tels que définis dans la formule générale (I) selon la revendication 1,
avec un carbonate de formule générale (III) 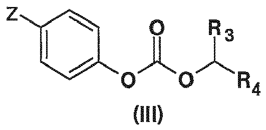 dans laquelle Z représente un atome d'hydrogène ou un groupe nitro, R₃ et R₄ sont tels que définis dans la formule générale (I) selon la revendication 1,
en présence d'une base, dans un solvant à une température comprise entre la température ambiante et la température de reflux du solvant.

7. Procédé de préparation d'un composé de formule (I) selon l'une quelconque des revendications 1 à 5, comprenant l'étape consistant à faire réagir un composé de formule générale (Ia) 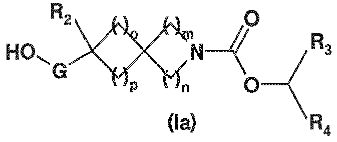 dans laquelle R₂, R₃, R₄, m, n, o et p sont tels que définis dans la formule générale (I) selon la revendication 1, et G représente une partie du groupe A tel que défini dans la formule générale (I), à savoir une liaison covalente;
avec soit un dérivé alcool de formule générale R₁OH (IV), dans laquelle R₁ est tel que défini dans la formule générale (I) selon la revendication 1, en utilisant les conditions de réaction de Mitsunobu;
soit avec un dérivé halogéné de formule générale R₁X (IVa), dans laquelle R₁ est tel que défini dans la formule générale (I) selon la revendication 1, et X représente un atome de fluor, de chlore, de brome ou d'iode en utilisant les réactions de substitution nucléophiles aromatiques, hétéroaromatiques ou de O-arylation, O-hétéroarylation de Buchwald.

8. Procédé de préparation d'un composé de formule (I) selon l'une quelconque des revendications 1 à 5, dans laquelle R₁ représente un groupe R₅ substitué notamment par un groupe R₆ de type C₁₋₆-alkyle, C₃₋₇-cycloalkyle ou C₃₋₇-cycloalkyle-C₁₋₃-alkylène, ou par un groupe R₇ tel que défini dans la formule générale (I) selon la revendication 1, comprenant l'étape consistant à réaliser une réaction de couplage, catalysée au moyen d'un métal de transition, sur le composé de formule générale (Ib), 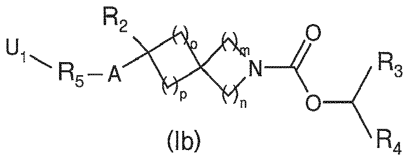 dans laquelle A, R₂, R₃, R₄, R₅, m, n, o et p sont tels que définis dans la formule générale (I) selon la revendication 1 et U₁ représente un atome de chlore, de brome, d'iode ou un groupement triflate, U₁ étant dans la position où l'on souhaite introduire le groupe R₆ ou R₇:
- soit par une réaction de type Suzuki, par exemple au moyen d'un acide boronique d'alkyle, de cycloalkyle, d'aryle ou d'hétéroaryle,
- soit selon une réaction de type Stille, par exemple en utilisant un dérivé tri-alkylstanneux d'aryle ou d'hétéroaryle
- soit par une réaction de type Negishi, par exemple en utilisant un dérivé zincate d'halogénure d'alkyle, de cycloalkyle, d'aryle ou d'hétéroaryle.

9. Composé de formule (III) : 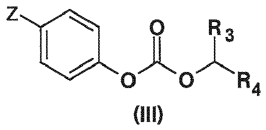 dans laquelle Z représente un atome d'hydrogène ou un groupe nitro, R₃ est tel que défini dans la formule (I) et R₄ représente un groupe 3-méthylcarbamoyl-isoxazol-5-ylméthyle.

10. Composé de formule générale (Ia) : 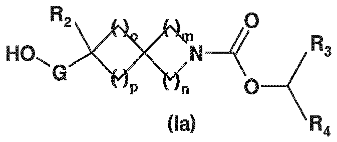 dans laquelle R₂, R₃, R₄, m, n, o et p sont tels que définis dans la revendication 1, et G représente une partie du groupe A tel que défini dans la formule générale (I), à savoir une liaison covalente.

11. Composé de formule générale (IIa) : 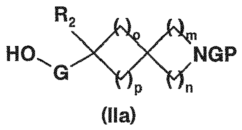 dans laquelle R₂ est tel que défini dans la formule générale (I) de la revendication 1, o et p représentent 1, m et n représentent 1 ou 2, mais m et n ne représentent pas ensemble la valeur 2, G représente une partie du groupe A tel que défini dans la formule générale (I), à savoir une liaison covalente, et GP représente un groupe protecteur tel qu'un Boc (tert-butyoxyocarbonyl), un Cbz (benzyloxycarbonyl, un benzyle ou un benzhydryle.

12. Composé de formule générale (II) : 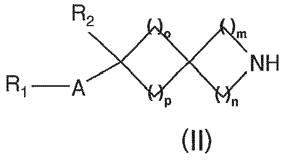 dans laquelle R₁, R₂, m, n, o et p sont tels que définis dans la revendication 1, A représente un atome d'oxygène, étant donné que R₁ n'est pas un groupe fluorophényl.

13. Composé de formule générale (IIe): 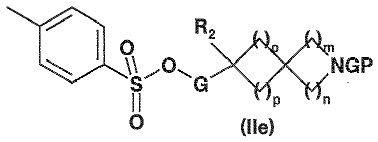 dans laquelle R₂ est tel que défini dans la revendication 1, o et p représentent 1, m et n représentent 1 ou 2, mais m et n ne représentent pas ensemble la valeur 2, G représente une partie du groupe A tel que défini dans la formule générale (I), à savoir une liaison covalente, et GP représente un groupe protecteur tel qu'un Boc (tert-butyoxyocarbonyl), un Cbz (benzyloxycarbonyl, un benzyle ou un benzhydryle.

14. Composé de formule (I) selon l'une quelconque des revendications 1 à 5, à l'état de base ou de sel d'addition à un acide pharmaceutiquement acceptable, pour son utilisation comme médicament.

15. Composition pharmaceutique contenant au moins un composé de formule (I) selon l'une quelconque des revendications 1 à 5, à l'état de base ou de sel d'addition à un acide pharmaceutiquement acceptable et éventuellement un ou plusieurs excipients pharmaceutiquement acceptables.

16. Utilisation d'un composé de formule (I) selon l'une quelconque des revendications 1 à 5, à l'état de base ou de sel d'addition à un acide pharmaceutiquement acceptable, pour la préparation d'un médicament destiné à prévenir ou à traiter une pathologie dans laquelle les cannabinoïdes endogènes et/ou tous autres substrats métabolisés par l'enzyme FAAH, sont impliqués.

17. Utilisation d'un composé de formule (I) selon l'une quelconque des revendications 1 à 5, à l'état de base ou de sel d'addition à un acide pharmaceutiquement acceptable, pour la préparation d'un médicament destiné à prévenir ou à traiter les douleurs aiguës ou chroniques de type neurogène, les douleurs aiguës ou chroniques associées aux maladies inflammatoires, les douleurs aiguës ou chroniques périphériques, les vertiges, les vomissements, les nausées, les troubles du comportement alimentaire, les pathologies neurologiques et psychiatriques, les maladies neuro-dégénératives aiguës ou chroniques, l'épilepsie, les troubles du sommeil, les maladies cardiovasculaires, l'ischémie rénale, les cancers, les désordres du système immunitaire, les maladies allergiques, les maladies infectieuses parasitaires , virales ou bactériennes, les maladies inflammatoires, l'ostéoporose, les affections oculaires, les affections pulmonaires, les maladies gastro-intestinales, l'incontinence urinaire ou l'inflammation vésicale.

## Patentansprüche

1. Verbindung mit der Formel (I) 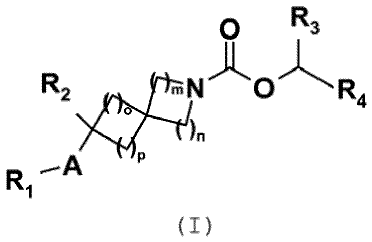 worin
R₂ für ein Wasserstoffatom, Fluoratom oder eine Hydroxyl-, Cyano-, Trifluormethyl-, C₁₋₆-Alkyl-, C₁₋₆-Alkoxygruppe, NR₈R₉ steht;
m, n, o und p den Wert 1 haben oder p und o den Wert 1 haben, n und m den Wert 2 haben, oder n, o und p den Wert 1 haben und m den Wert 2 hat; A für ein Sauerstoffatom steht;
R₁ für eine Gruppe R₅, gegebenenfalls substituiert mit einer oder mehreren Gruppen R₆ und/oder R₇, steht;
R₅ für eine Gruppe, ausgewählt aus einem Phenyl, Pyridinyl, Pyridazinyl, Pyrimidinyl, Pyrazinyl, Triazinyl, Naphthalinyl, Chinolinyl, Isochinolinyl, Phtalazinyl, Chinazolinyl, Chinoxalinyl, Cinnolinyl, Naphthyridinyl, steht;
R₆ für ein Halogenatom, eine Cyanogruppe, -CH₂CN, Nitro, Hydroxyl, C₁₋₆-Alkyl, C₁₋₆-Alkoxy, C₁₋₆-Thioalkyl, C₁₋₆-Haloalkyl, C₁₋₆-Haloalkoxy, C₁₋₆-Halothioalkyl, C₃₋₇-Cycloalkyl, C₃₋₇-Cycloalkyl-C₁₋₃-alkylen, C₃₋₇-Cycloalkyl-C₁₋₃-alkylen-O-, NR₈R₉, NR₈COR₉, NR₈CO₂R₉, NR₈SO₂R₉, NR₈SO₂NR₈R₉, COR₈, CO₂R₈, CONR₈R₉, SO₂R₈, SO₂NR₈R₉ oder -O-(C₁₋₃-Alkylen)-O-steht;
R₇ für eine Gruppe, ausgewählt aus Phenyl, Pyridinyl, Pyridazinyl, Pyrimidinyl, Pyrazinyl, Triazinyl, steht; wobei die Gruppe(n) R₇ mit einer oder mehreren Gruppen R₆, gleich oder verschieden voneinander, substituiert sein kann/können;
R₃ für ein Wasserstoffatom, Fluoratom, eine C₁₋₆-Alkylgruppe oder eine Trifluormethylgruppe steht;
R₄ für eine Gruppe, ausgewählt aus einem Thiazolyl, einem Triazolyl, einem Oxazolyl und einem Isoxazolyl steht; wobei diese Gruppe nicht substituiert oder mit einer oder mehreren C₁₋₆-Alkylgruppe(n) oder CONR₈R₉ substituiert ist;
R₈ und R₉ unabhängig voneinander für ein Wasserstoffatom oder eine C₁₋₆-Alkylgruppe stehen;
in Form einer Base oder eines Säureadditionssalzes.

2. Verbindung mit der Formel (I) nach Anspruch 1, **dadurch gekennzeichnet, dass** R₂ für ein Wasserstoffatom steht,
in Form einer Base oder eines Säureadditionssalzes.

3. Verbindung mit der Formel (I) nach Anspruch 1 oder 2, **dadurch gekennzeichnet, dass** R₁ für eine Gruppe R₅ steht, die nicht substituiert oder mit einer oder mehreren Gruppen R₆ und/oder R₇ substituiert ist;
R₅ für eine Phenyl-, Naphthalinyl- oder Isochinolinylgruppe steht;
R₆ für ein Halogenatom, insbesondere ein Fluor- oder Chloratom, oder eine C₁₋₆-Haloalkylgruppe, insbesondere eine Trifluormethylgruppe, oder eine C₁₋₆-Alkoxygruppe, insbesondere eine Ethoxygruppe steht;
R₇ für ein Phenyl steht, das mit einer oder mehreren Gruppen R₆, gleich oder verschieden voneinander, substituiert sein kann, in Form einer Base oder eines Säureadditionssalzes.

4. Verbindung mit der Formel (I) nach einem der Ansprüche 1 bis 3, **dadurch gekennzeichnet, dass** R₃ für ein Wasserstoffatom steht, in Form einer Base oder eines Säureadditionssalzes.

5. Verbindung mit der Formel (I), ausgewählt aus:
Thiazol-4-ylmethyl-2-(4-chlorphenoxy)-7-azaspiro[3.5]nonan-7-carboxylat;
2-Methyl-2H-[1,2,4]triazol-3-ylmethyl-2-(4-chlorphenoxy)-7-aza-spiro[3.5]nonan-7-carboxylat und sein Hydrochlorid;
3-Methylcarbamoyl-isoxazol-5-ylmethyl-2-(4-chlorphenoxy)-7-aza-spiro[3.5]nonan-7-carboxylat;
3-Carbamoyl-isoxazol-5-ylmethyl-2-(4-chlorphenoxy)-7-aza-spiro[3.5]nonan-7-carboxylat;
3-Methylcarbamoyl-isoxazol-5-ylmethyl-2-(3-trifluormethylphenoxy)-7-aza-spiro[3.5]nonan-7-carboxylat;
3-Carbamoyl-isoxazol-5-ylmethyl-2-(3-trifluormethyl-phenoxy)-7-aza-spiro[3.5]nonan-7-carboxylat;
3-Methylcarbamoyl-isoxazol-5-ylmethyl-2-(4'-fluorbiphenyl-3-yloxy)-7-aza-spiro[3.5]nonan-7-carboxylat;
3-Methylcarbamoyl-isoxazol-5-ylmethyl-2-(7-ethoxynaphthalin-2-yloxy)-7-aza-spiro[3.5]nonan-7-carboxylat;
3-Carbamoyl-isoxazol-5-ylmethyl-2-(3-trifluormethylphenoxy)-6-aza-spiro[3.4]octan-6-carboxylat und sein Hydrochlorid (Isomere I + II);
3-Carbamoyl-isoxazol-5-ylmethyl-2-(3-trifluormethylphenoxy)-6-aza-spiro[3.4]octan-6-carboxylat (Isomer I der Verbindung Nr. 9);
3-Carbamoyl-isoxazol-5-ylmethyl-2-(3-trifluormethylphenoxy)-6-aza-spiro[3.4]octan-6-carboxylat (Isomer II der Verbindung Nr. 9);
3-Carbamoyl-isoxazol-5-ylmethyl-2-(4'-fluorbiphenyl-3-yloxy)-7-aza-spiro[3.5]nonan-7-carboxylat;
3-Carbamoyl-isoxazol-5-ylmethyl-2-(7-ethoxynaphthalin-2-yloxy)-7-aza-spiro[3.5]nonan-7-carboxylat;
4-Carbamoyl-oxazol-2-ylmethyl-2-(3-trifluormethylphenoxy)-6-aza-spiro[3.4]octan-6-carboxylat (Isomere I + II);
4-Methylcarbamoyl-oxazol-2-ylmethyl-2-(3-trifluormethylphenoxy)-6-aza-spiro[3.4]octan-6-carboxylat (Isomere I + II);
3-Carbamoyl-isoxazol-5-ylmethyl-2-(4-chlornaphthalin-1-yloxy)-7-aza-spiro[3.5]nonan-7-carboxylat;
3-Methylcarbamoyl-isoxazol-5-ylmethyl-2-(4-chlornaphthalin-1-yloxy)-7-aza-spiro[3.5]nonan-7-carboxylat;
3-Carbamoyl-isoxazol-5-ylmethyl-2-(4'-fluorbiphenyl-4-yloxy)-7-aza-spiro[3.5]nonan-7-carboxylat;
3-Methylcarbamoyl-isoxazol-5-ylmethyl-2-(4'-fluorbiphenyl-4-yloxy)-7-aza-spiro[3.5]nonan-7-carboxylat;
4-Methylcarbamoyl-thiazol-2-ylmethyl-2-(4'-fluorbiphenyl-3-yloxy)-7-aza-spiro[3.5]nonan-7-carboxylat;
3-Methylcarbamoyl-isoxazol-5-ylmethyl-2-(isochinolin-7-yloxy)-7-aza-spiro[3.5]nonan-7-carboxylat;
3-Methylcarbamoyl-isoxazol-5-ylmethyl-2-(4-chlor-3-fluorphenoxy)-7-aza-spiro[3.5]nonan-7-carboxylat;
3-Methylcarbamoyl-isoxazol-5-ylmethyl-6-(4-chlor-3-fluorphenoxy)-2-aza-spiro[3.3]heptan-2-carboxylat;
3-Carbamoyl-isoxazol-5-ylmethyl-6-(4-chlor-3-fluorphenoxy)-2-aza-spiro[3.3]heptan-2-carboxylat;
3-Methylcarbamoyl-isoxazol-5-ylmethyl-6-(4'-fluorbiphenyl-4-yloxy)-2-aza-spiro[3.3]heptan-2-carboxylat;
3-Carbamoyl-isoxazol-5-ylmethyl-2-(4-chlor-3-fluorphenoxy)-6-aza-spiro[3.4]octan-6-carboxylat (Isomere I + II);
3-Methylcarbamoyl-isoxazol-5-ylmethyl-2-(4-chlorphenoxy)-6-aza-spiro[3.4]octan-6-carboxylat (Isomere I + II);
3-Carbamoyl-isoxazol-5-ylmethyl-2-(4'-fluorbiphenyl-4-yloxy)-6-aza-spiro[3.4]octan-6-carboxylat (Isomere I + II);
3-Methylcarbamoyl-isoxazol-5-ylmethyl-2-(4'-fluorbiphenyl-4-yloxy)-6-aza-spiro[3.4]octan-6-carboxylat (Isomere I + II);
3-Carbamoyl-isoxazol-5-ylmethyl-2-(4-chlornaphthalin-1-yloxy)-6-aza-spiro[3.4]octan-6-carboxylat (Isomere I + II);
3-Methylcarbamoyl-isoxazol-5-ylmethyl-2-(4-chlornaphthalin-1-yloxy)-6-aza-spiro[3.4]octan-6-carboxylat (Isomere I + II);
3-Methylcarbamoyl-isoxazol-5-ylmethyl-2-(4'-fluorbiphenyl-4-yloxy)-6-aza-spiro[3.4]octan-6-carboxylat (Isomer I der Verbindung 29);
3-Methylcarbamoyl-isoxazol-5-ylmethyl-2-(4'-fluorbiphenyl-4-yloxy)-6-aza-spiro[3.4]octan-6-carboxylat (Isomer II der Verbindung 29).

6. Verfahren zur Herstellung einer Verbindung mit der Formel (I) nach einem der Ansprüche 1 bis 5, umfassend den Schritt des Umsetzens eines Amins mit der allgemeinen Formel (II), 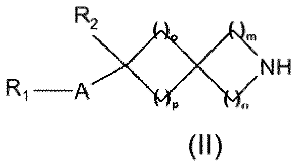 worin A, R₁, R₂, m, n, o und p die in der allgemeinen Formel (I) nach Anspruch 1 definierte Bedeutung haben,
mit einem Carbonat der allgemeinen Formel (III) 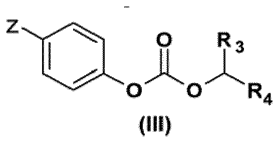 worin Z für ein Wasserstoffatom oder eine Nitrogruppe steht, R₃ und R₄ die in der allgemeinen Formel (I) nach Anspruch 1 definierte Bedeutung haben,
in Gegenwart einer Base, in einem Lösemittel bei einer Temperatur im Bereich zwischen der Umgebungstemperatur und der Rückflusstemperatur des Lösemittels.

7. Verfahren zur Herstellung einer Verbindung mit der Formel (I) nach einem der Ansprüche 1 bis 5, umfassend den Schritt des Umsetzens einer Verbindung mit der allgemeinen Formel (Ia), 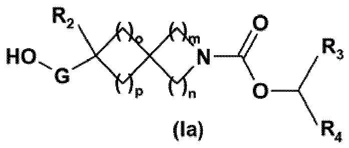 worin R₂, R₃, R₄, m, n, o und p die in der allgemeinen Formel (I) nach Anspruch 1 definierte Bedeutung haben, und G für einen Teil der Gruppe A steht, wie in der allgemeinen Formel (I) definiert, d. h. eine kovalente Bindung;
entweder mit einem Alkoholderivat mit der allgemeinen Formel R₁OH (IV), worin R₁ die in der allgemeinen Formel (I) nach Anspruch 1 definierte Bedeutung hat, unter Verwendung der Bedingungen der Mitsunobu-Reaktion;
oder mit einem Halogenderivat mit der allgemeinen Formel R₁X (IVa), worin R₁ die in der allgemeinen Formel (I) nach Anspruch 1 definierte Bedeutung hat, und X für ein Fluor-, Chlor-, Brom - oder Iodatom steht, unter Verwendung der Buchwald-Reaktionen zur nukleophilen aromatischen, heteroaromatischen Substitution oder O-Arylierung, O-Heteroarylierung.

8. Verfahren zur Herstellung einer Verbindung mit der Formel (I) nach einem der Ansprüche 1 bis 5, worin R₁ für eine Gruppe R₅ steht, die insbesondere mit einer Gruppe R₆ vom Typ C₁₋₆-Alkyl, C₃₋₇-Cycloalkyl oder C₃₋₇-Cycloalkyl-C₁₋₃-alkylen, oder mit einer Gruppe R₇, wie in der allgemeinen Formel (I) nach Anspruch 1 definiert, substituiert ist, umfassend den Schritt der Durchführung einer Kopplungsreaktion, die mithilfe eines Übergangsmetalls katalysiert wird, an der Verbindung mit der allgemeinen Formel (Ib), 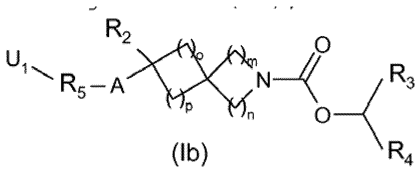 worin A, R₂, R₃, R₄, R₅, m, n, o und p sind, wie in der allgemeinen Formel (I) nach Anspruch 1 definiert, und U₁ für ein Chlor-, Brom-, Iodatom oder eine Triflat-Gruppe steht, wobei U₁ in der Position steht, an der die Gruppe R₆ oder R₇ eingeführt werden soll:
- oder durch eine Reaktion vom Typ Suzuki, zum Beispiel mithilfe einer Alkyl-, Cycloalkyl-, Aryl- oder Heteroarylboronsäure,
- oder gemäß einer Reaktion vom Typ Stille, zum Beispiel unter Verwendung eines Aryl- oder Heteroaryl-tri-Zinntetraalkylderivats,
- oder durch eine Reaktion vom Typ Negishi, zum Beispiel unter Verwendung eines Alkyl-, Cycloalkyl-, Aryl- oder Heteroarylhalogenidzinkatderivats,

9. Verbindung mit der Formel (III): 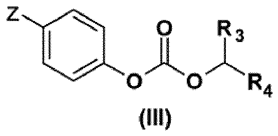 worin Z für ein Wasserstoffatom oder eine Nitrogruppe steht, R₃ die in der Formel (I) definierte Bedeutung hat, und R₄ für eine 3-(Methylcarbamoyl)-isoxazol-5-ylmethylgruppe steht.

10. Verbindung mit der allgemeinen Formel (Ia) : 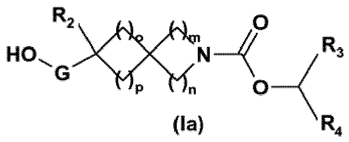 worin R₂, R₃, R₄, m, n, o und p die in Anspruch 1 definierte Bedeutung haben, und G für einen Teil der Gruppe A steht, wie in der allgemeinen Formel (I) definiert, d. h. eine kovalente Bindung.

11. Verbindung mit der allgemeinen Formel (IIa): 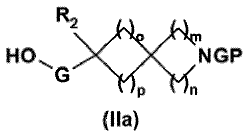 worin R₂ die in der allgemeinen Formel (I) von Anspruch 1 definierte Bedeutung hat, o und p für 1 stehen, m und n für 1 oder 2 stehen, aber m und n zusammen nicht für den Wert 2 stehen, G für einen Teil der Gruppe A steht, wie in der allgemeinen Formel (I) definiert, nämlich eine kovalente Bindung, und GP für eine Schutzgruppe, wie z. B. Boc (tert-Butyoxyocarbonyl), Cbz (Benzyloxycarbonyl, Benzyl oder Benzhydryl, steht.

12. Verbindung mit der allgemeinen Formel (II): 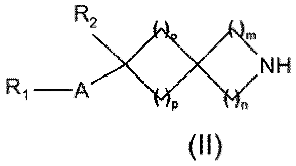 worin R₁, R₂, m, n, o und p die in Anspruch 1 definierte Bedeutung haben, A für ein Sauerstoffatom steht, wenn R₁ keine Fluorphenylgruppe ist.

13. Verbindung mit der allgemeinen Formel (IIe): 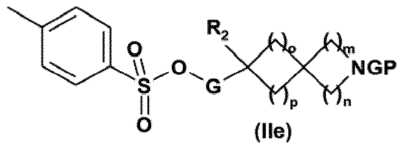 worin R₂ die in Anspruch 1 definierte Bedeutung hat, o und p für 1 stehen, m und n für 1 oder 2 stehen, aber m und n zusammen nicht für den Wert 2 stehen, G für einen Teil der Gruppe A steht, wie in der allgemeinen Formel (I) definiert, d. h. eine kovalente Bindung, und GP für eine Schutzgruppe, wie z. B. Boc (tert-Butyoxyocarbonyl), Cbz (Benzyloxycarbonyl, Benzyl oder Benzhydryl, steht.

14. Verbindung mit der Formel (I) nach einem der Ansprüche 1 bis 5 in Form einer Base oder eines Additionssalzes einer pharmazeutisch verträglichen Säure zur Verwendung als Medikament.

15. Pharmazeutische Zusammensetzung, enthaltend mindestens eine Verbindung mit der Formel (I) nach einem der Ansprüche 1 bis 5 in Form einer Base oder eines Additionssalzes einer pharmazeutisch verträglichen Säure und gegebenenfalls einem oder mehreren pharmazeutisch verträglichen Exzipienten.

16. Verwendung einer Verbindung mit der Formel (I) nach einem der Ansprüche 1 bis 5 in Form einer Base oder eines Additionssalzes einer pharmazeutisch verträglichen Säure zur Herstellung eines Medikaments, das dazu bestimmt ist, eine Erkrankung zu verhindern oder zu behandeln, an der die endogenen Cannabinoide und/oder alle anderen Substrate, die durch das Enzym FAAH verstoffwechselt werden, beteiligt sind.

17. Verwendung einer Verbindung mit der Formel (I) nach einem der Ansprüche 1 bis 5 in Form einer Base oder eines Additionssalzes einer pharmazeutisch verträglichen Säure zur Herstellung eines Medikaments, das dazu bestimmt ist, chronische oder akute neurogene Schmerzen, akute oder chronische Schmerzen, die mit Entzündungskrankheiten verbunden sind, akute oder chronische periphere Schmerzen, Schwindelzustände, Erbrechen, Übelkeiten, Störungen des Essverhaltens, neurologische und psychiatrische Erkrankungen, akute oder chronische neurodegenerative Krankheiten, Epilepsie, Schlafstörungen, Herz-Kreislauf-Krankheiten, Nierenischämie, Krebsformen, Störungen des Immunsystems, allergische Krankheiten, parasitäre, virale oder bakterielle Infektionskrankheiten, Entzündungskrankheiten, Osteoporose, Augenleiden, Lungenleiden, Magen-Darm-Krankheiten, Harninkontinenz oder Blasenentzündung zu verhindern oder zu behandeln.

## Claims

1. Compound corresponding to the formula (I): 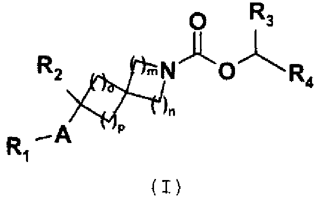 in which
R₂ represents a hydrogen or fluorine atom or a hydroxyl, cyano, trifluoromethyl, C₁₋₆ alkyl, C₁₋₆ alkoxy or NR₈R₉ group;
m, n, o and p have the value 1, or else p and o have the value 1, and n and m have the value 2, or else n, o and p have the value 1 and m has the value 2;
A represents an oxygen atom;
R₁ represents a group R₅ optionally substituted with one or more groups R₆ and/or R₇;
R₅ represents a group selected from a phenyl, pyridinyl, pyridazinyl, pyrimidinyl, pyrazinyl, triazinyl, naphthalenyl, quinolinyl, isoquinolinyl, phthalazinyl, quinazolinyl, quinoxalinyl, cinnolinyl or naphthyridinyl group;
R₆ represents a halogen atom, a cyano, -CH₂CN, nitro, hydroxyl, C₁₋₆-alkyl, C₁₋₆-alkoxy, C₁₋₆-thioalkyl, C₁₋₆-haloalkyl, C₁₋₆-haloalkoxy, C₁₋₆-halothioalkyl, C₃₋₇-cycloalkyl, C₃₋₇-cycloalkyl-C₁₋₃-alkylene, C₃₋₇-cycloalkyl-C₁₋₃-alkylene-O-, NR₈R₉, NR₈COR₉, NR₈CO₂R₉, NR₈SO₂R₉, NR₈SO₂NR₈R₉, COR₈, CO₂R₈, CONR₈R₉, SO₂R₈, SO₂NR₈R₉ or -O-(C₁₋₃-alkylene)-O- group;
R₇ represents a group selected from a phenyl, pyridinyl, pyridazinyl, pyrimidinyl, pyrazinyl or triazinyl group, the group or group(s) R₇ possibly being substituted with one or more groups R₆ which are identical to or different from one other;
R₃ represents a hydrogen or fluorine atom, a C₁₋₆-alkyl group or a trifluoromethyl group;
R₄ represents a group selected from a thiazolyl, triazolyl, oxazolyl and isoxazolyl; this group being unsubstituted or substituted with one or more C₁₋₆-alkyl or CONR₈R₉ groups;
R₈ and R₉ independently of each other represent a hydrogen atom or a C₁₋₆-alkyl group;
in the form of the base or an acid addition salt.

2. Compound of formula (I) according to Claim 1, **characterized in that** R₂ represents a hydrogen atom;
in the form of the base or an acid addition salt.

3. Compound of formula (I) according to Claim 1 or 2, **characterized in that** R₁ represents a group R₅, unsubstituted or substituted with one or more groups R₆ and/or R₇;
R₅ represents a phenyl, naphthalenyl or isoquinolinyl group;
R₆ represents a halogen atom, more particularly a fluorine or chlorine atom, or a C₁₋₆-haloalkyl, more particularly trifluoromethyl, group or a C₁₋₆-alkoxy group, more particularly an ethoxy group; and
R₇ represents a phenyl which may be substituted with one or more groups R₆ which are identical to or different from one another, in the form of the base or an acid addition salt.

4. Compound of formula (I) according to any one of Claims 1 to 3, **characterized in that** R₃ represents a hydrogen atom, in the form of the base or an acid addition salt.

5. Compound of formula (I) selected from:
Thiazol-4-ylmethyl 2-(4-chlorophenoxy)-7-aza-spiro-[3.5]nonane-7-carboxylate;
2-methyl-2H-[1,2,4]triazol-3-ylmethyl 2-(4-chlorophenoxy)-7-aza-spiro[3.5]nonane-7-carboxylate and the hydrochloride thereof;
3-methylcarbamoyl-isoxazol-5-ylmethyl 2-(4-chlorophenoxy)-7-aza-spiro[3.5]nonane-7-carboxylate;
3-carbamoyl-isoxazol-5-ylmethyl 2-(4-chlorophenoxy)-7-aza-spiro[3.5]nonane-7-carboxylate;
3-methylcarbamoyl-isoxazol-5-ylmethyl 2-(3-trifluoromethylphenoxy)-7-aza-spiro[3.5]nonane-7-carboxylate;
3-carbamoyl-isoxazol-5-ylmethyl 2-(3-trifluoromethylphenoxy)-7-aza-spiro[3.5]nonane-7-carboxylate;
3-methylcarbamoyl-isoxazol-5-ylmethyl 2-(4'-fluorobiphenyl-3-yloxy)-7-aza-spiro[3.5]nonane-7-carboxylate;
3-methylcarbamoyl-isoxazol-5-ylmethyl 2-(7-ethoxynaphthalen-2-yloxy)-7-aza-spiro[3.5]nonane-7-carboxylate;
3-carbamoyl-isoxazol-5-ylmethyl 2-(3-trifluoromethylphenoxy)-6-aza-spiro[3.4]octane-6-carboxylate and the hydrochloride thereof (isomers I + II);
3-carbamoyl-isoxazol-5-ylmethyl 2-(3-trifluoromethylphenoxy)-6-aza-spiro[3.4]octane-6-carboxylate (isomer I of compound No.9);
3-carbamoyl-isoxazol-5-ylmethyl 2-(3-trifluoromethylphenoxy)-6-aza-spiro[3.4]octane-6-carboxylate (isomer II of compound No.9);
3-carbamoyl-isoxazol-5-ylmethyl 2-(4'-fluorobiphenyl-3-yloxy)-7-aza-spiro[3.5]nonane-7-carboxylate;
3-carbamoyl-isoxazol-5-ylmethyl 2-(7-ethoxynaphthalen-2-yloxy)-7-aza-spiro[3.5]nonane-7-carboxylate;
4-carbamoyl-oxazol-2-ylmethyl 2-(3-trifluoromethylphenoxy)-6-aza-spiro[3.4]octane-6-carboxylate (isomers I + II);
4-methylcarbamoyl-oxazol-2-ylmethyl 2-(3-trifluoromethylphenoxy)-6-aza-spiro[3.4]octane-6-carboxylate (isomers I + II);
3-carbamoyl-isoxazol-5-ylmethyl 2-(4-chloronaphthalen-1-yloxy)-7-aza-spiro[3.5]nonane-7-carboxylate;
3-methylcarbamoyl-isoxazol-5-ylmethyl 2-(4-chloronaphthalen-1-yloxy)-7-aza-spiro[3.5]nonane-7-carboxylate;
3-carbamoyl-isoxazol-5-ylmethyl 2-(4'-fluorobiphenyl-4-yloxy)-7-aza-spiro[3.5]nonane-7-carboxylate;
3-methylcarbamoyl-isoxazol-5-ylmethyl 2-(4'-fluorobiphenyl-4-yloxy)-7-aza-spiro[3.5]nonane-7-carboxylate;
4-methylcarbamoyl-thiazol-2-ylmethyl 2-(4'-fluorobiphenyl-3-yloxy)-7-aza-spiro[3.5]nonane-7-carboxylate;
3-methylcarbamoyl-isoxazol-5-ylmethyl 2-(isoquinolin-7-yloxy)-7-aza-spiro[3.5]nonane-7-carboxylate;
3-methylcarbamoyl-isoxazol-5-ylmethyl 2-(4-chloro-3-fluorophenoxy)-7-aza-spiro[3.5]nonane-7-carboxylate;
3-methylcarbamoyl-isoxazol-5-ylmethyl 6-(4-chloro-3-fluorophenoxy)-2-aza-spiro[3.3]heptane-2-carboxylate;
3-carbamoyl-isoxazol-5-ylmethyl 6-(4-chloro-3-fluorophenoxy)-2-aza-spiro[3.3]heptane-2-carboxylate;
3-methylcarbamoyl-isoxazol-5-ylmethyl 6-(4'-fluorobiphenyl-4-yloxy)-2-aza-spiro[3.3]heptane-2-carboxylate;
3-carbamoyl-isoxazol-5-ylmethyl 2-(4-chloro-3-fluorophenoxy)-6-aza-spiro[3.4]octane-6-carboxylate (isomers I + II);
3-methylcarbamoyl-isoxazol-5-ylmethyl 2-(4-chlorophenoxy)-6-aza-spiro[3.4]octane-6-carboxylate (isomers I + II);
3-carbamoyl-isoxazol-5-ylmethyl 2-(4'-fluorobiphenyl-4-yloxy)-6-aza-spiro[3.4]octane-6-carboxylate (isomers I + II);
3-methylcarbamoyl-isoxazol-5-ylmethyl 2-(4'-fluorobiphenyl-4-yloxy)-6-aza-spiro[3.4]octane-6-carboxylate (isomers I + II);
3-carbamoyl-isoxazol-5-ylmethyl 2-(4-chloronaphthalen-1-yloxy)-6-aza-spiro[3.4]octane-6-carboxylate (isomers I + II);
3-methylcarbamoyl-isoxazol-5-ylmethyl 2-(4-chloronaphthalen-1-yloxy)-6-aza-spiro[3.4]octane-6-carboxylate (isomers I + II);
3-methylcarbamoyl-isoxazol-5-ylmethyl 2-(4'-fluorobiphenyl-4-yloxy)-6-aza-spiro[3.4]octane-6-carboxylate (isomer I of compound 29);
3-methylcarbamoyl-isoxazol-5-ylmethyl 2-(4'-fluorobiphenyl-4-yloxy)-6-aza-spiro[3.4]octane-6-carboxylate (isomer II of compound 29).

6. Process for preparation of a compound of formula (I) according to any one of Claims 1 to 5, **characterized in that** it comprises the stage consisting in reacting an amine of general formula (II), 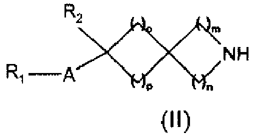 in which A, R₁, R₂, m, n, o and p are as defined in the general formula (I) according to Claim 1,
with a carbonate of general formula (III), 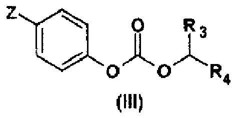 in which Z represents a hydrogen atom or a nitro group, R₃ and R₄ are as defined in the general formula (I) according to Claim 1,
in the presence of a base, in a solvent at a temperature lying between ambient temperature and the reflux temperature of the solvent.

7. Process for preparation of a compound of formula (I) according to any one of Claims 1 to 5, comprising the stage consisting in reacting a compound of general formula (Ia) 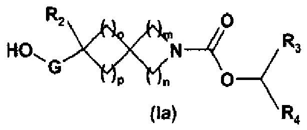 in which R₂, R₃, R₄, m, n, o and p are as defined in the general formula (I) according to Claim 1 and G represents a part of the group A as defined in the general formula (I) namely either a covalent bond;
with either an alcohol derivative of general formula R₁OH (IV), in which R₁ is as defined in the general formula (I) according to Claim 1, using the Mitsunobu reaction conditions;
or with a halogenated derivative of general formula R₁X (IVa) in which R₁ is as defined in the general formula (I) according to Claim 1, and X represents a fluorine, chlorine, bromine or iodine atom using aromatic or heteroaromatic nucleophilic substitution, or O-arylation or Buchwald O-heteroarylation reactions.

8. Process for preparation of a compound of formula (I) according to any one of Claims 1 to 5, in which R₁ represents a group R₅ substituted in particular with a group R₆ of the C₁₋₆-alkyl, C₃₋₇-cycloalkyl or C₃₋₇-cycloalkyl-C₁₋₃-alkylene type, or with a group R₇ as defined in the general formula (I) according to Claim 1, **characterized in that** it comprises the stage consisting in performing a coupling reaction, catalysed by means of a transition metal, on the compound of general formula (Ib), 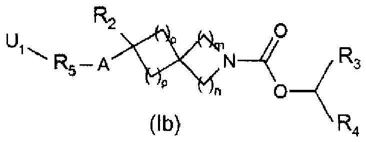 in which A, R₂, R₃, R₄, R₅, m, n, o and p are as defined in the general formula (I) according to Claim 1 and U₁ represents a chlorine, bromine or iodine atom or a triflate group, U₁ being in the position where it is desired to introduce the group R₆ or R₇ :
- either by a Suzuki type reaction, for example by means of an alkyl, cycloalkyl, aryl or heteroaryl boronic acid,
- or by a Stille type reaction, for example using an aryl or heteroaryl tri-alkyltin derivative
- or by a Negishi type reaction, for example using an alkyl, cycloalkyl, aryl or heteroaryl halide zincate derivative.

9. Compound of formula (III) 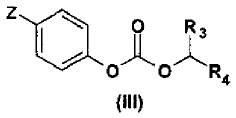 in which Z represents a hydrogen atom or a nitro group, R₃ is as defined in the formula (I) and R₄ represents a 3-(methylcarbamoyl)isoxazol-5-ylmethyl group.

10. Compound of general formula (Ia): 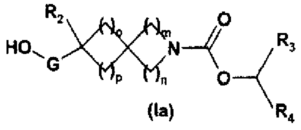 in which R₂, R₃, R₄, m, n, o and p are as defined in Claim 1 and G represents a part of the group A as defined in the general formula (I), namely a covalent bond.

11. Compound of general formula (IIa): 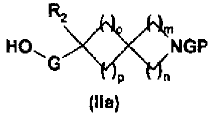 in which R₂ is as defined in the general formula (I) of Claim 1, o and p represent 1, m and n represent 1 or 2, but m and n do not together represent the value 2, G represents a part of the group A as defined in the general formula (I), namely a covalent bond, and GP represents a protective group such as a Boc (*tert-*butyloxycarbonyl), Cbz (benzyloxycarbonyl), benzyl or benzhydryl.

12. Compound of general formula (II): 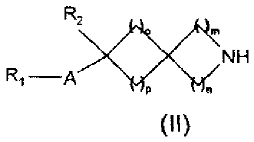 in which R₁, R₂, m, n, o and p are as defined in Claim 1, and A represents an oxygen atom, it being given that R₁ is not a fluorophenyl group.

13. Compound of general formula (IIe): 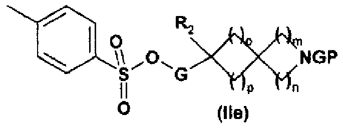 in which R₂ is as defined in Claim 1, o and p represent 1, m and n represent 1 or 2, but m and n do not together represent the value 2, G represents a part of the group A as defined in the general formula (I), namely a covalent bond, and GP represents a protective group such as a Boc (tert-butyloxycarbonyl), Cbz (benzyloxycarbonyl), benzyl or benzhydryl.

14. Compound of general formula (I) according to any one of Claims 1 to 5, in the form of the base or addition salt to a pharmaceutically acceptable acid, for the use thereof as a medicament.

15. Pharmaceutical composition containing at least one compound of formula (I) according to any one of Claims 1 to 5, in the form of the base or addition salt to a pharmaceutically acceptable acid, and optionally one or more pharmaceutically acceptable excipients.

16. Use of a compound of formula (I) according to any one of Claims 1 to 5, in the form of the base or addition salt to a pharmaceutically acceptable acid, for the preparation of a medicament intended to prevent or to treat a pathology in which the endogenous cannabinoids and/or any other substrates metabolized by the enzyme FAAH are involved.

17. Use of a compound of formula (I) according to any one of Claims 1 to 5, in the form of the base or addition salt to a pharmaceutically acceptable acid, for the preparation of a medicament intended to prevent or to treat acute or chronic pain of neurogenic type, acute or chronic pain associated with inflammatory diseases, acute or chronic peripheral pain, vertigo, vomiting, nausea, eating disorders, neurological and psychiatric pathologies, acute and chronic neurodegenerative diseases, epilepsy, sleep disorders, cardiovascular diseases, renal ischaemia, cancers, immune system disorders, allergic diseases, parasitic, viral or bacterial infectious diseases, inflammatory diseases, osteoporosis, ocular complaints, gastrointestinal diseases, urinary incontinence or inflammation of the bladder.
